(19) Europäisches Patentamt / European Patent Office / Office européen des brevets

(11) **EP 4 549 920 A1**

(12) **EUROPEAN PATENT APPLICATION**

(43) Date of publication:
**07.05.2025 Bulletin 2025/19**

(21) Application number: **24209142.9**

(22) Date of filing: **28.10.2024**

(51) International Patent Classification (IPC):
**G01N 25/18** $^{(2006.01)}$   **G01N 33/00** $^{(2006.01)}$

(52) Cooperative Patent Classification (CPC):
**G01N 25/18; G01N 33/0078**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.11.2023 IT 202300023040**

(71) Applicants:
• **Università della Calabria**
  **87036 Rende (CS) (IT)**
• **Vega Energia S.r.l.**
  **87036 Rende (CS) (IT)**

(72) Inventors:
• **ARCURI, Natale**
  **Rende (CS) (IT)**
• **NICOLETTI, Francesco**
  **Rende (CS) (IT)**
• **BRUNO, Roberto**
  **Rende (CS) (IT)**
• **BEVILACQUA, Piero**
  **Rende (CS) (IT)**
• **CARPINO, Cristina**
  **Rende (CS) (IT)**
• **PANZA, Pietro**
  **Rende (CS) (IT)**
• **BASTANZA, Pasqualino**
  **Rende (CS) (IT)**

(74) Representative: **Dall'Olio, Christian et al**
**INVENTION S.r.l.**
**Via delle Armi, 1**
**40137 Bologna (IT)**

(54) **MEASUREMENT OF THE SURFACE TO SURFACE THERMAL CONDUCTANCE OF AN OPAQUE BUILDING COMPONENT**

(57)    A method for in situ measurement of the surface to surface thermal conductance of an opaque component (O) comprising steps of providing at least a first heater and/or cooler (17, 17'), a second heater and/or cooler (27, 27'), placing a first hollow object (10) and a second hollow object (20) on the opaque component (O), obtaining a first volume (10V) and a second volume (20V), repeatedly measuring a heat flow or, alternatively, a thermal power, maintaining both the first volume (10V) and the second volume (20V) at a first test temperature $T_a$ and at a second test temperature $T_b$ determining heat flows or thermal powers for calclating corrective coefficients of the heat flow or the thermal power with the first volume (10V) and the second volume (20V) respectively maintained at $T_c$ and $T_d$. A method for measuring the thermal capacitance (C) and systems for measuring the properties of an opaque component (O).

FIG. 1

EP 4 549 920 A1

## Description

The present invention concerns the technical sector relating to instruments and methods for measurement, in particular for the in situ analysis or investigation, i.e. in the work place, of the opaque components of the structures with investigations or analyses that use thermal means. In greater detail, the invention relates to method, a system and a computer program for measurement of the thermal conductance and/or the thermal capacitance of an opaque component.

Measurements at the work site of an opaque component of a building are useful in determining real thermal performances: for example with the aim of investigating the thermal characteristics of an opaque component with an unknown stratigraphy, of verifying the existence of the desired heat resistance and/or evaluating any effects of degradation.

In situ measurement of a representative volume of the opaque component avoids assessing the thermal performance on the basis of a theoretical stratigraphy, or one detected in the field, avoids the use of invasive or destructive methods, such as making small holes or taking samples, and enables obtaining a value that is close to that of the real building.

Standard ISO 9869-1:2014 describes a method using a heat flow meter for the in situ measurement of some heat transmission properties of flat components of buildings such as heat resistance, surface to surface thermal conductance, total heat resistance and the heat transmission between ambients, when the ambient temperatures are known on both sides. These properties can be measured without having to priorly know the stratigraphy of the opaque component. The method of standard ISO 9869-1:2014 includes two approaches of data analysis, a first approach which takes account of the average of the measurements carried out for a minimum of three days and a second dynamic approach. Though the method is devised for components constituted by opaque layers perpendicular to the heat flow, standard ISO 9869-1:2014 suggests use thereof also for almost-homogeneous layers though the results require a dynamic analysis in the case that the building subjected to the test has a good level of thermal capacitance. The precision of the results obtainable with these methods is not comparable with that of the measurements in the laboratory which include encapsulating a specimen of the opaque component between two controlled-temperature chambers. Examples of methods with laboratory-taken measurements are presented in standard ISO 8990:1994 and in Zarr, R.R., Burch, D.M., Faison, T.K., and Arnold, C.E. Thermal Resistance Measurements of Well-Insulated and Superinsulated Residential Walls Using a Calibrated Hot Box. Journal of Thermal Insulation. 1987; 10(3):197-218 <doi:10.1177/109719638701000306>. Further, the method of the first approach in standard ISO 9869-1:2014 is particularly negatively affected by fluctuations of temperatures.

Other examples of methods sanctioned the standards are described in standard ASTM C115: a first method is alike to the method discussed in standard ISO 9869-1:2014 while a second method is applicable only on walls made up of a single homogeneous material.

As the method of laboratory measurements are not applicable to in situ measurements, completely changing the configuration of the test and the impact on the opaque component to be measured, and since the methods for in situ measurement are subject to errors that are difficult to predict, depending on the value of the same properties that are to be measured, overtime, numerous methods and alternative systems of measurement have been developed. Document KR20200101727A illustrates a method that uses heaters, a cooler, temperature sensors and a heat flow meter and which includes the observation of the response to a heat pulse.

Document EP2769190A4 describes a method for improving a measurement of heat flow in which the temperature of a space delimited by a hollow body and by the opaque component is maintained in equilibrium with the temperature of the outside ambient air on the same side of the opaque component.

Document WO2012049417A2 estimates the heat resistance and the thermal capacitance by applying temperature sensors on the two sides of an opaque component and in a screened ambient and using a simulation using a method based on finite differences. In this case too the wall is modelled as if it were composed of a single layer of homogeneous material, as occurs in standard ASTM C1155 or as discussed in the article by Cucumo, M., de Rosa, A., Ferraro, V., Kaliakatsos, D., and Marinelli, V. (2006). A method for the experimental evaluation in-situ of the wall conductance. Energy and Buildings, 38(3), 238-244 <https://doi.org/10.1016/J.ENBUILD.2005.06.005>.

Patent application IT201700148950, converted into Utility Model IT202000001282, illustrates a system comprising two insulated objects and cables to be laid, with the openings facing in the same direction, at the portion of opaque component on which the measurement is to be made, a heat flow meter on both walls, four sensors for detecting the surface temperatures on the two sides of the opaque component, a thermostat resistance for maintaining a temperature constant internally of the space delimited by an insulated and hollow object and the opaque component is itself a data recorder.

Document WO2016150856A1 describes application of two enclosed volumes on opposite sides of an opaque component and of controlling, by heating or cooling, the difference of temperature between the two enclosed volumes so as to assess the heat transmission on the basis of the temperatures measured, of the area facing onto the closed volume and of the energy supplied. With the aim of limiting the disturbances due to the difference of temperature between the enclosed volumes and the surrounding ambients, document WO2016150856A1 suggests enclosing the first enclosed volumes in further enclosed volumes at a controlled temperature. The distorting effects of the heat flow between the surfaces are thus reduced but with an increase of components, required space for the test, and costs; without counting that the distorting effects tend to cancel each other out only in the case of very large enclosed volumes.

The article authored by Nicoletti, F., Cucumo, M., & Arcuri, N. (2023). Evaluating the Accuracy of In-situ Methods for Measuring Wall Thermal Conductance: A Comparative Numerical Study. Energy and Buildings, 113095 <https://doi.org/10.1016/J.ENBUILD.2023.113095> proposed an assessment of some methods for in situ measurement on various types of walls and in different ambient conditions. What emerges is a large gap between the values measured of the thermal properties and the comparative values obtained from numerical simulations.

Still today there is a perceived need for a method and an apparatus for in situ measurement which guarantees lower error levels in relation to the required complexity, as well as the costs for the system. In particular the need is perceived for an approach which enables taking account of the distorting effects on the in situ measurements of the ambient temperatures on both sides of the opaque component and, possibly, the distorting effects on the in situ measurements of the fluctuations of the ambient temperatures in general present on one or both sides.

The present invention intends to obviate one or more drawbacks of the solutions of the prior art, while remaining within an experimental context characterised by errors in measurement that are not insignificant and by the variability of the test conditions, so that the technical expert in the sector is used to assessing the validity of an approach not on a single case but on the basis of the trend in repeated tests, as proposed, for example, in the article by Nicoletti et alii cited in the foregoing.

A first aim of the present invention is to provide a method or a computer program or a system useful for the in situ measurement of the surface to surface thermal conductance and/or of the thermal capacitance of an opaque component, each of which enables taking account of the distorting effects of the environmental temperatures on both sides of the opaque component.

An aim of some embodiments of the present invention is to further improve the characterisation of the opaque component, especially of opaque components that are particularly insulated and/or which have a plurality of layers that are different to one another, i.e. with a non-homogenous stratigraphy.

A further aim of some embodiments of the present invention is to simplify the system required for in situ measurement or reducing the costs with respect to the existing solutions, while maintaining a correction of the distorting effects of the ambient temperatures at the sides of the opaque component.

A still further aim of some embodiments of the present invention is to operate with non-flat opaque components, i.e. with surfaces that are significantly non-homogeneous, such as for example occurs in the case of walls consisting of natural and unclad stone.

A still further aim of some embodiments of the present invention is to simplify and/or to make more rapid the characterisation of the opaque component, for example by enabling the use of systems having smaller dimensions and/or by enabling obtaining more thermal properties of the opaque component with a same operating sequence.

A further aim of some embodiments of the present invention is to provide an easy-to-use in situ system with respect to the reliability of the measurements it will make.

These and other aims, which will be obvious to the expert in the sector from a reading of the following text, are attained by means of a first method for the in situ measurement of the surface to surface thermal conductance $\Lambda$ of a volume of an opaque component of a building, of a second method for in situ measurement of the thermal capacitance of a volume of an opaque component of a building, of a computer program for measuring the surface to surface thermal conductance $\Lambda$ and/or the thermal capacitance of a volume of an opaque component of a building, of a first system for measuring the surface to surface thermal conductance $\Lambda$ and/or the thermal capacitance of a volume of an opaque component of a building or of a second system for carrying out the first method according to the contents of the claims.

In accordance with the teachings of the present document, the first method comprises steps of

- providing at least a first heater and/or cooler;
- providing at least a second heater and/or cooler;
- placing a first hollow object and a second hollow object on opposite sides of an opaque component in such a way as to enclose lateral surfaces, opposite one another, of a volume of opaque component and which delimit, together with the opaque component, respectively a first volume and a second volume heated and/or cooled respectively by the at least a first heater and/or cooler and by the at least a second heater and/or cooler;
- measuring a plurality of times,

    measuring at least a heat flow through at least a part of a respective surface of the lateral surfaces so as to obtain at least a series of measurements of heat flow relative to a surface of the lateral surfaces or, alternatively, measuring a thermal power supplied by the at least a first heater and/or cooler and a thermal power supplied by the at least a second heater and/or cooler, so as to obtain series of measurements of thermal power supplied respectively to the first volume and to the second volume;

- maintaining $T_c$ and $T_d$, i.e. maintaining the first volume and the second volume at a respective test temperature $T_c$ and $T_d$ different between the first volume and the second volume, for a predetermined time interval t and/or at least until when the variation between time-successive measurements of a series of the at least a series of measurements of heat flow or of a series of the series of measurements of thermal power is lower than a desired value d ;
- determining at least a value q of heat flow or of thermal power with the first volume and the second volume at the respective test temperature $T_c$ and $T_d$, each value q of the at least a value q being determined by a measurement, at the end of the step of maintaining $T_c$ and $T_d$, of a respective series of the at least a series of measurements of heat flow or by a representative value of the trend over time of measurements of a respective series of the series of measurements of thermal power in a time interval, within the step of maintaining $T_c$ and $T_d$;

The first method advantageously also comprises steps of:

- maintaining $T_a$, i.e. maintaining the first volume and the second volume at a first test temperature $T_a$, equal between the first volume and the second volume, for a first predetermined time interval $t_1$ and/or at least until when the variation between time-successive measurements of a series of the at least a series of measurements of heat flow or of a series of the series of measurements of thermal power is lower than a first desired value $d_1$;
- determining at least a first value $q_a$ of heat flow or of thermal power with the first volume and the second volume at the first test temperature $T_a$, each value of the at least a first value $q_a$ being determined by a first measurement, at the end of the step of maintaining $T_a$, of a respective series of the at least a series of measurements of heat flow or by a first representative value of the trend over time of measurements of a respective series of the series of measurements of thermal power in a first time interval, within the step of maintaining $T_a$;
- maintaining $T_b$, maintaining the first volume and the second volume at a second test temperature $T_b$, equal between the first volume and the second volume, for a second predetermined time interval $t_2$ and/or at least until when the variation between time-successive measurements of a series of the at least a series of measurements of heat flow or of a series of the series of measurements of thermal power is lower than a second desired value $d_2$;
- determining at least a second value $q_b$ of heat flow or of thermal power with the first volume and the second volume at the second test temperature $T_b$, each value of the at least a second value $q_b$ being determined by a second measurement, at the end of the step of maintaining $T_b$, of a respective series of the at least a series of measurements of heat flow or by a second representative value of the trend over time of measurements of a respective series of the series of measurements of thermal power in a second time interval, within the step of maintaining $T_b$;
- calculating corrective coefficients of the at least a value q of heat flow or of thermal power, on the basis of the at least a first value $q_a$ and of the at least a second value $q_b$, the corrective coefficients correcting the effects of a first outside temperature $T_{1e}$ at the side of the opaque component where the first hollow object is placed and externally of the first hollow object and the effects of a second outside temperature $T_{2e}$ at the side of the opaque component where the second hollow object is placed and externally of the second hollow object;
- determining a corrected value q' of heat flow or of thermal power with the first volume and the second volume at the respective test temperature $T_c$ and $T_d$ on the basis of the at least a value q of heat flow or of thermal power and of the corrective coefficients;
- calculating the surface to surface thermal conductance $\Lambda$ using the corrected value q' of heat flow or of thermal power.

In accordance with the teachings of the present document, the second method comprises the steps of:

- providing at least a first heater and/or cooler;
- providing at least a second heater and/or cooler;
- placing a first hollow object and a second hollow object on opposite sides of an opaque component in such a way as to enclose lateral surfaces, opposite one another, of a volume of opaque component and which delimit, together with the opaque component, respectively a first volume and a second volume heated and/or cooled, respectively by the at least a first heater and/or cooler and by the at least a second heater and/or cooler;
- measuring a heat flow, continuously or at time intervals, through at least a part of a first surface of the lateral surfaces facing towards the first hollow object and a heat flow through at least a part of a second surface of the lateral surfaces facing towards the second hollow object so as to obtain at least a first series of measurements of heat flow relative to the first surface and a second series of measurements of heat flow relative to the second surface;
- maintaining $T_a$, maintaining the first volume and the second volume at a first test temperature $T_a$, equal between the first volume and the second volume, for a first predetermined time interval $t_1$ and/or at least until when the variation between time-successive measurements of a series of the first series of measurements and the second series of measurements is lower than a first desired value $d_1$;
- maintaining $T_b$, maintaining the first volume and the second volume at a second test temperature $T_b$, equal between the first volume and the second volume, until when the variation between time-successive measurements of a series of the first series of measurements and the second series of measurements is lower than second desired value $d_2$;

- calculating the thermal capacitance C of the volume of opaque component on the basis of the first series of measurements and of the second series of measurements carried out between the start and the end of the step of maintaining $T_b$.

The step of maintaining $T_b$ begins at the end of the step of maintaining $T_a$.

In accordance with the teachings of the present document, the computer program comprises instructions for carrying out at least some steps of the first method.

In accordance with the teachings of the present document, the first system comprises a first unit, a second unit, one or more systems of acquisition of the detections and at least a control unit.

The first unit comprises a first hollow object comprising at least a wall, a seal and an opening, at least an air temperature sensor, at least a first heater and/or cooler and a heat flow meter.

The second unit comprises a second hollow object comprising at least a second wall, a second seal and a second opening, at least a second air temperature sensor, at least a second heater and/or cooler heats and a second heat flow meter.

The first unit and the second unit are on opposite sides of an opaque component with the first hollow object facing towards the second hollow object positioned in such a way as to enclose lateral surfaces, mutually opposite, of a volume of an opaque component and so as to delimit, together with the opaque component, respectively a first volume and a second volume.

The seal surrounds the opening and is interposed between the at least a wall and the opaque component so as to guarantee the air seal between the at least a wall and the opaque component.

The second seal surrounds the second opening and is interposed between the at least a second wall and the opaque component so as to guarantee the air seal between the at least a second wall and the opaque component.

The at least an air temperature sensor is located internally of the first volume.

The at least a second air temperature sensor is located internally of the second volume.

The at least a first heater and/or cooler heats and/or cools the first volume.

The at least a second heater and/or cooler heats and/or cools the second volume.

The at least a control unit receives information or detections from at least a sensor of the at least an air temperature sensor and from at least a sensor of the at least a second air temperature sensor and controls the at least a first heater and/or cooler and the at least a second heater and/or cooler.

The heat flow meter is applied on a first surface and the second heat flow meter is applied on a second surface, opposite the first surface, of the lateral surfaces of the volume of opaque component.

In accordance with the teachings of the present document, the second system comprises a first unit, a second unit, one or more systems of acquisition of the detections, at least a control unit and the computer program.

The first unit comprises a first hollow object comprising at least a wall, a seal and an opening, at least an air temperature sensor, an air temperature sensor, at least a first heater and/or cooler and first means for measuring a heat flow or a thermal power supplied by the at least a first heater and/or cooler.

The second unit comprises a second hollow object comprising at least a second wall, a second seal and a second opening, at least a second air temperature sensor, a second air temperature sensor and at least a second heater and/or cooler.

The first unit and the second unit are on opposite sides of an opaque component with the first hollow object facing towards the second hollow object positioned in such a way as to enclose lateral surfaces, mutually opposite, of a volume of an opaque component and so as to delimit, together with the opaque component, respectively a first volume and a second volume.

The seal surrounds the opening and is interposed between the at least a wall and the opaque component so as to guarantee the air seal between the at least a wall and the opaque component.

The second seal surrounds the second opening and is interposed between the at least a second wall and the opaque component so as to guarantee the air seal between the at least a second wall and the opaque component.

The at least an air temperature sensor is located internally of the first volume.

The air temperature sensor is located externally of the first hollow object.

The at least a second air temperature sensor is located internally of the second volume.

The second air temperature sensor is located externally of the second hollow object.

The at least a first heater and/or cooler heats and/or cools the first volume.

The at least a second heater and/or cooler heats and/or cools the second volume.

The at least a control unit receives information or detections from at least a sensor of the at least an air temperature sensor and from at least a sensor of the at least a second air temperature sensor and controls the at least a first heater and/or cooler and the at least a second heater and/or cooler.

The first means measure a heat flow on the surface facing towards the first hollow object of the mutually opposite lateral surfaces of the volume of opaque component or measure a thermal power supplied by the at least a first heater and/or cooler; in the case where the first means measure the thermal power, the second unit comprises second means for measuring a thermal power supplied by the at least a second heater and/or cooler.

Specific embodiments of the invention will be described in the following part of the present description, according to what is set down in the claims and with the aid of the accompanying drawings, in which:

- figure 1 is a schematic perspective view of an embodiment of a packaging system according to the invention with a first hollow object of a first unit and a second hollow object of a second unit illustrated distant from an opaque component of a building for a readier comprehension;

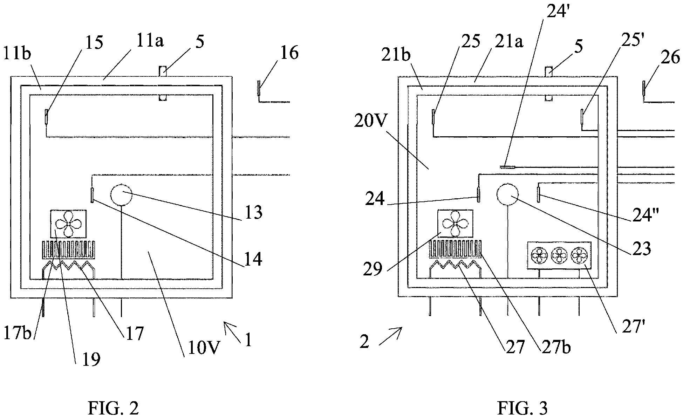

- figure 2 is a schematic view of part of an embodiment of the first unit and also representative of the second unit which might be realised thus;

- figure 3 is a schematic view of part of an embodiment of the second unit, more complete with respect to the embodiment visible in figure 2, in this case too the first unit might be realised as illustrated in this figure;

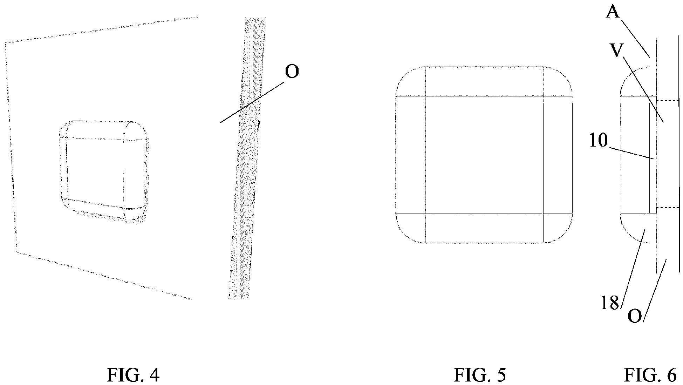

- figure 4 shows part of an embodiment of the first unit or the second unit positioned on an opaque component of a building;

- figure 5 shows a frontal view of the unit of figure 4;

- figure 6 is a lateral view of the first unit realised according to figure 4;

- figure 7 is a schematic view of an embodiment of the second unit and also representative of the first unit which might be realised thus;

- figure 8 is a schematic view of an embodiment of the first unit, more complete with respect to the embodiment visible in figure 7, in this case too the second unit might be realised as illustrated in this figure;

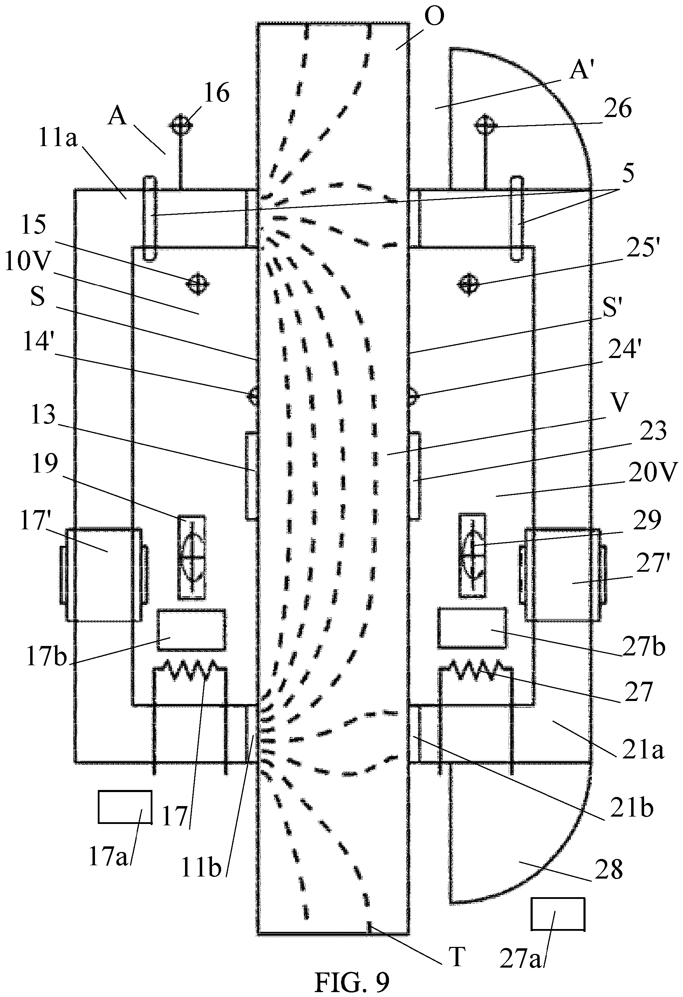

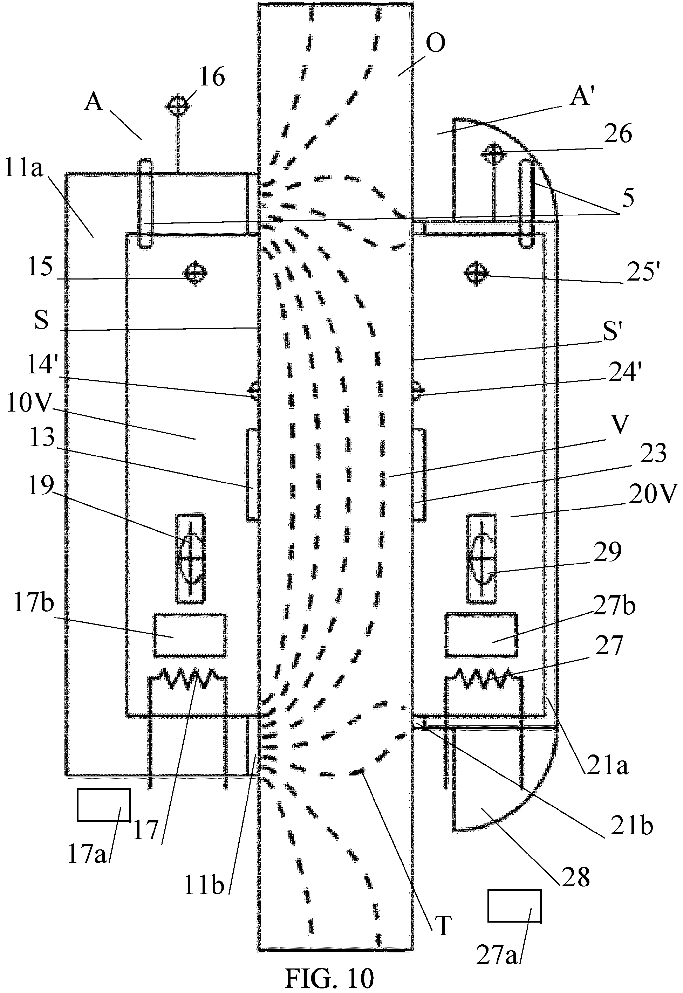

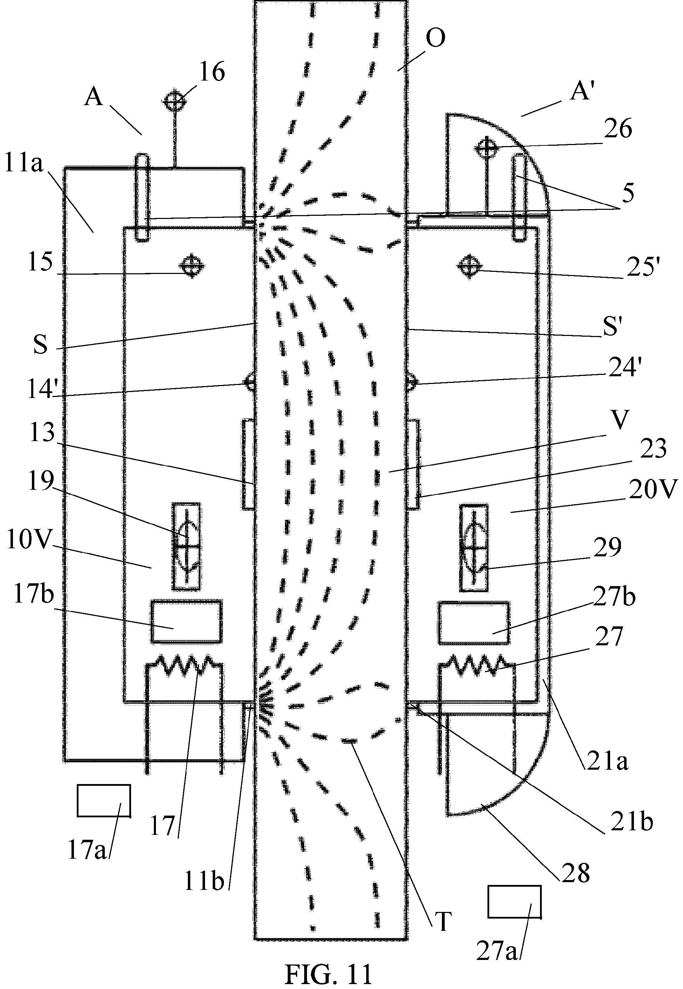

- each of figures 9, 10 and 11 show a schematic section view of a portion of a respective embodiment of a system, complete with a representation of isotherms internally of an opaque component, in order to describe the non-one-dimensional nature of the heat flow;

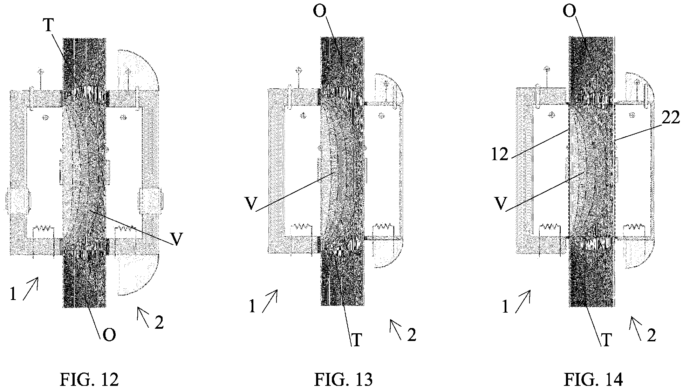

- figures 12, 13 and 14 show figures 9, 10 and 11 in greyscale;

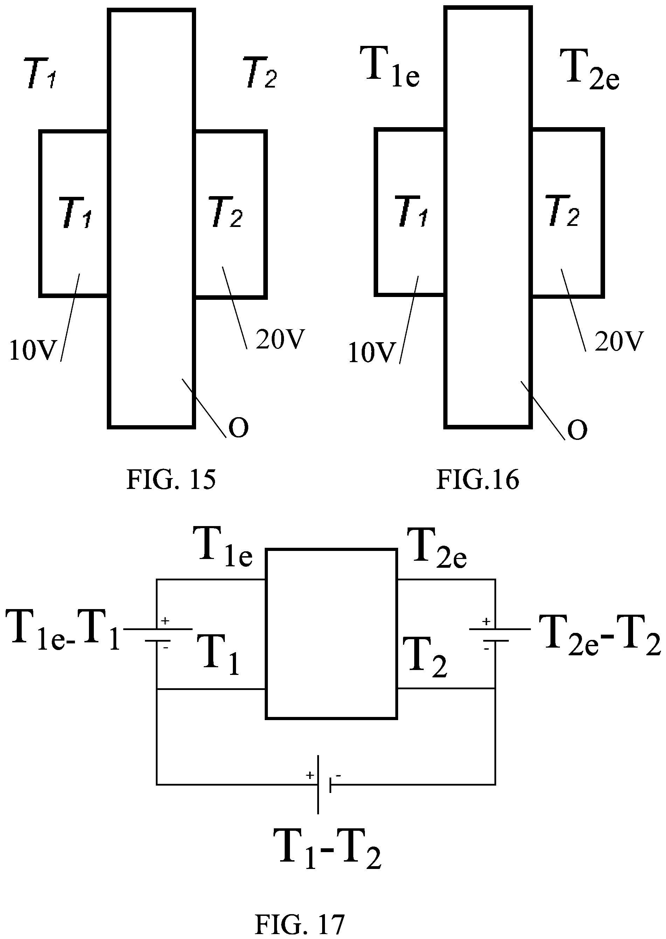

- figure 15 schematically shows a theoretical case of measurement of the surface to surface thermal conductance $\Lambda$ of an opaque component of a building;

- figure 16 is an actual case of figure 15;

- figure 17 is an electrical diagram of the real case of figure 16;

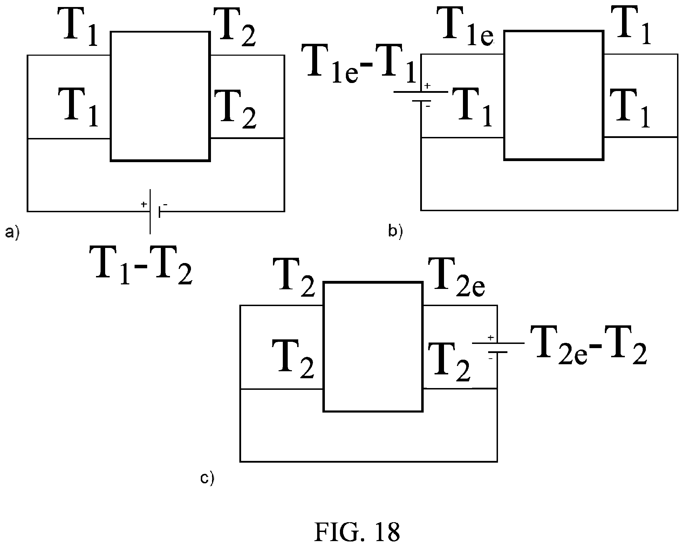

- figure 18 shows the electrical diagram of figure 17 after the application of the superposition principle of the effects.

With reference to the appended drawings, reference V denotes a volume of an opaque component (O) of a building. The references are included to facilitate comprehension of the invention, but do not limit the scope of protection.

An embodiment of a method for carrying out the in situ measurement of the surface to surface thermal conductance $\Lambda$ of a volume (V) of an opaque component (O) of a building comprises steps of:

- providing at least a first heater and/or cooler (17, 17');
- providing at least a second heater and/or cooler (27, 27');
- placing a first hollow object (10) and a second hollow object (20) on opposite sides of an opaque component (O) in such a way as to enclose lateral surfaces (S, S'), mutually opposite, of a volume (V) of an opaque component (O) and which delimit, together with the opaque component (O), respectively a first volume (10V) and a second volume (20V) heated and/or cooled respectively by the at least a first heater and/or cooler (17, 17') and by the at least a second heater and/or cooler (27, 27');
- measuring a plurality of times,

measuring at least a heat flow through at least a part of a respective surface of the lateral surfaces (S, S') so as to obtain at least a series of measurements of heat flow relative to a surface of the lateral surfaces (S, S') or, alternatively,

measuring a thermal power supplied by the at least a first heater and/or cooler (17, 17') is measured as well as a thermal power supplied by the at least a second heater and/or cooler (27, 27'), so as to obtain series of measurements of thermal power supplied respectively to the first volume (10V) and to the second volume (20V);

- maintaining $T_c$ and $T_d$, maintaining the first volume (10V) and the second volume (20V) at a respective test temperature $T_c$ and $T_d$ different between the first volume (10V) and the second volume (20V), for a predetermined time interval $t$ and/or at least until when the variation between time-successive measurements of a series of the at least a series of measurements of heat flow or of a series of the series of measurements of thermal power lower that a desired value $d$ ;

- determining at least a value q of heat flow or of thermal power with the first volume (10V) and the second volume (20V) at the respective test temperature $T_c$ and $T_d$, each value of the at least a value q being determined by a first measurement, at the end of the step of maintaining $T_c$ and $T_d$, of a respective series of the at least a series of measurements of heat flow or by a representative value of the trend over time of measurements of a respective series of the series of measurements of thermal power in a time interval, within the step of maintaining $T_c$ and $T_d$,

- maintaining $T_a$, maintaining the first volume (10V) and the second volume (20V) at a first test temperature $T_a$, equal between the first volume (10V) and the second volume (20V), for a first predetermined time interval $t_1$ and/or at least until when the variation between time-successive measurements of a series of the at least a series of measurements of heat flow or of a series of the series of measurements of thermal power is lower than a first desired value $d_1$;

- determining at least a first value $q_a$ of heat flow or of thermal power with the first volume (10V) and the second volume (20V) at the first test temperature $T_a$, each value of the at least a first value $q_a$ being determined by a first measurement, at the end of the step of maintaining $T_a$, of a respective series of the at least a series of measurements of heat flow or by a first representative value of the trend over time of measurements of a respective series of the series of measurements of thermal power in a first time interval, within the step of maintaining $T_a$;

- maintaining $T_b$, maintaining the first volume (10V) and the second volume (20V) at a second test temperature $T_b$, equal between the first volume (10V) and the second volume (20V), for a second predetermined time interval $t_2$ and/or at least until when the variation between time-successive measurements of a series of the at least a series of measurements of heat flow or of a series of the series of measurements of thermal power is lower than a second desired value $d_2$;

- determining at least a second value $q_b$ of heat flow or of thermal power with the first volume (10V) and the second volume (20V) at the second test temperature $T_b$, each value of the at least a second value $q_b$ being determined by a second measurement, at the end of the step of maintaining $T_b$, of a respective series of the at least a series of measurements of heat flow or by a second representative value of the trend over time of measurements of a respective series of the series of measurements of thermal power in a second time interval, within the step of maintaining $T_b$;

- calculating corrective coefficients of the at least a value q of heat flow or of thermal power, on the basis of the at least a first value $q_a$ and of the at least a second value $q_b$, the corrective coefficients correcting the effects of a first outside temperature $T_{1e}$ at the side of the opaque component (O) where the first hollow object (10) is placed and externally of the first hollow object (10) and the effects of a second outside temperature $T_{2e}$ at the side of the opaque component (O) where the second hollow object (20) is placed and externally of the second hollow object (20);

- determining a corrected value q' of heat flow or of thermal power with the first volume (10V) and the second volume (20V) at the respective test temperature $T_c$ and $T_d$ on the basis of the at least a value q of heat flow or of thermal power and of the corrective coefficients;

- calculating the surface to surface thermal conductance $\Lambda$ using the corrected value q' of heat flow or of thermal power.

The method enables, in a non-destructive way, measuring representative values of the distorting effects due to the temperatures of ambients on both sides of the opaque component (O). In particular the measurement is non-invasive and does not cause damage to the opaque component (O).

In the sector of buildings, i.e. of existing buildings, an opaque component is an element with no parts that are transparent to solar radiation, for example an external vertical wall, an internal vertical wall, a horizontal slab or a roof. It is known to the expert in the sector that the measurement of a volume (V) relates to the whole of the opaque component (O) in the hypothesis that the opaque component (O) is or is near to being homogeneous, not in terms of stratigraphy but of repetition of the same stratigraphy in space. Processes have therefore been developed, some of these the object of a specific standard, directed at guaranteeing the correct positioning of the of first hollow object (10) and of the second hollow object (20); for example to avoid the measurement of a part of opaque component (O) crossed by pipes of the air conditioning system. These processes, though of interest in the carrying out of the methods described herein, are referred to in passing only because they are known and not relevant to the purposes of the present invention.

Heat conductivity indicates the behaviour of a substance, in stationary conditions, in transmitting heat energy by heat conduction, i.e. without contributions by convectivity and heat radiation. In the case of a non-homogeneous material, this attitude to transmission of heat energy in stationary conditions and without the contributions of supply on the two sides and heat radiation, is known as thermal conductance.

In stationary conditions, i.e. with a single-directional heat flow between the lateral surfaces (S, S'), surface to surface thermal conductance $\Lambda$ is a scalar magnitude equal to the ratio between the heat flow, or density of heat flow, and the difference of the temperatures of the lateral surfaces (S, S'). The theoretical measurement of the surface to surface thermal conductance $\Lambda$ requires two lateral surfaces (S, S'), flat and parallel to one another, a condition which is generally only approximated by buildings.

In the in situ measurement with the first hollow object (10) and the second hollow object (20) the temperature field installed internally of the opaque component (O), even in the hypothesis of having maintained $T_c$ and $T_d$ over a very long time interval $t$, is in any case three-dimensional for various reasons:

- heat exchange by radiation, for example owing to the incident solar radiation on the surface of the opaque component (O) free from the second hollow object (20);
- convective heat exchange, for example with different convective heat exchange coefficients between the first volume (10V) and the second volume (20V) or between the inside and outside of the first hollow object (10);
- interruption or reduction of the heat flow in the interface between the first hollow object (10) or the second hollow object (20) and the opaque component (O), for example in a case where the first hollow object (10) is insulated this effect becomes more significant;
- difference between the first outside temperature $T_{1e}$ or the second outside temperature $T_{2e}$ and the temperature, respectively, internally of the first volume (10V) and internally of the second volume (20V).

The distorsions of the in situ measurement due to the first three reasons are not significant and/or a limitable, while the last reason represents the main source of distorsion.

By way of example: the distorsions due to the heat exchange by radiation can be reduced by the use of screens, as will be illustrated in the following; the distorsions due to the convective heat exchange can be reduced by realising conditions alike to the outside conditions internally of the first volume (10V) and the second volume (20V) with the use of ventilation and they are in any case insignificant; and the distorsions for the interruption or reduction of the heat flow can be reduced by reducing the thickness involved, as will be illustrated in the following, and they are in any case insignificant.

The distorsions of the in situ measurement due to the above can be reduced by increasing the dimensions of the first volume (10V) and the second volume (20V) and/or by performing measurements at the centre of the lateral surfaces (S, S'). These distorsions tend to be annulled only by infinite volumes; the increase in the volumes influences negatively on the applicability of the first hollow object (10) and the second hollow object (20) on an opaque component (O) as well as on the manageability of the system of measurement (100), also because, for in situ measurement, energy demands are higher. The solution proposed in WO2016150856A1 also does not obviate this drawback since externally of the outer chamber, which encloses the internal chamber, there are outside temperatures that are not controlled.

The distorsions mentioned above are evident even in the case of fixed or stationary conditions, i.e. in the case in which the first outside temperature $T_{1e}$ and the second outside temperature $T_{2e}$ are constant: in the case of oscillations it is preferable to use average values, as will be illustrated in the following.

On completion of the step of maintaining $T_a$ after a first time interval $t_1$ that is sufficiently long and/or after having reached variations lower than a first desired value $d_1$, there are almost stationary conditions and the heat flow between the first volume (10V) and the second volume (20V) through the volume (V) should be theoretically nil. The at least a first value $q_a$ is therefore mainly attributed to the distorsions due to the first outside temperature $T_{1e}$ and to the second outside temperature $T_{2e}$.

For this reason the measurement considered is the one detected at the end of the step.

The same reasoning is applied on completion of the step of maintaining $T_b$ and to the at least a second value $q_b$. The at least a first value $q_a$ and the at least a second value $q_b$ can then be used to resolve a system of equations deriving from the heat modelling of the test and for calculating corrective coefficients of the at least a value q of heat flow or of thermal power.

The following part of the description will describe useful formulas in a case where the step of calculating corrective coefficients takes place after two steps of maintaining the first volume (10V) and the second volume (20V) at a same temperature, which is considered to be a preferred embodiment both because it represents the simplest case and because it enables an eventual calculation of the thermal capacitance (C).

In any case the formulas used could be different, both should the step of calculating corrective coefficients involve further steps of maintaining the first volume (10V) and the second volume (20V) at a same temperature, and should a different heat model and/or other simplifications be applied.

In the case of the formulas treated in the following, the annulling of the theoretical heat flow at a first test

temperature $T_a$ and at a second test temperature $T_b$ is useful for resolving the system of equations deriving from the application of the superposition principle of the effects to the forcing mechanisms that act on the heat flow through the volume (V), as will be detailed in the following and as illustrated schematically in figure 18.

The method, initially developed for measurement of heat flow, has demonstrated its further applicability for measuring thermal power, although in this second case it is strongly recommended, indeed is necessary, to verify the reliability of the measurement by calculating and verifying a substantial equivalence between a first corrected value $q'_1$

and a second corrected value $q'_2$, each being calculated as described in the following.

The method is therefore described in terms of heat flow and in terms of thermal power, but the two options are not combinable, leading to the expression "or, alternatively," in the description of the step of measuring a plurality of times. In other words, the steps of starting from repeatedly measuring relate to the heat flow or, alternatively, to the thermal power.

Further, in the steps of determining at least a value, at least a first value and at least a second value to take account of the possibility of having one or two series of measurements, on one or both sides of the opaque component (O).

The measurements of thermal power are in general more economical and simple to realise, as they do not require a heat flow meter (13, 23) or like means for measurement, as well as finding advantageous application in the case of lateral surfaces (S, S') that are significantly nonhomogeneous, i.e. not flat, in which the application of a heat flow meter (13, 23) would be difficult and, in any case, not representative of the behaviour of the opaque component (O). The measurements of the heat flow enable the simplification of the measurement system (100) on one or both sides of the opaque component (O) as they can use on only heat flow meter (13, 23).

With only one single heat flow meter (13, 23), or like means for measurement, which with the measurements of thermal power it is not possible to measure the thermal capacitance (C) of the volume (V) of opaque component (O). Preferably, though not necessarily, in the step of measuring repeatedly, the measurements are continuous or at time intervals. In the second case it is preferable that two successive measurements are distanced by time from one another by between 15 and 45 minutes, more preferably 30 minutes.

The first hollow object (10) and the second hollow object (20) enable creating an ambient with a reduced or null mass exchange with the outside. As will be evidenced in the description of the systems (100) herein below, the first hollow object (10) and the second hollow object (20) typically have a parallelepiped shape with an open side and the other sides formed by insulated walls; at least one of these is generally perforated for the passage of wires for transmission of data or energy, though wireless modalities can be used, and for installation of a breather valve (5).

In the step of measuring a plurality of times, in the case of measuring at least a heat flow, the heat flow is measured through one or both the lateral surfaces (S, S'), preferably through a central part of one or both the lateral surfaces (S, S') so that the measurement is less affected by the variability of the test conditions. In the case where the measurements of heat flow relate to a single surface of the lateral surfaces (S, S') a series of measurements of heat flow will be obtained relative to the single surface, otherwise two.

The corrective coefficients enable taking account of the non-one-dimensional nature of the heat flow shown in figures from 12 to 14, due to the first outside temperature $T_{1e}$ and to the second outside temperature $T_{2e}$ which are not the same as the inside temperatures, respectively of the first volume (10V) and the second volume (20V); this difference between the real test conditions and the theoretical conditions, illustrated in the comparison between between figure 15 and figure 16.

In the step of repeatedly measuring, it is preferable to measure at least one heat flow as it does not suffer from the dispersions through the first hollow object (10) and the second hollow object (20) and because, in a case where it is measured through a part, and especially in the case that the part is central, the measurement is less affected by the variability of the test conditions. Numerical tests have shown how the measurements of the surface to surface thermal conductance $\Lambda$ obtained, in various conditions, with two series of measurements of heat flow, presented smaller errors.

Consequently, and preferably:

- in the step of measuring a plurality of times, at least a heat flow is measured through at least a part of a respective surface of the lateral surfaces (S, S') so as to obtain at least a series of measurements of heat flow relative to a surface of the lateral surfaces (S, S');
- in the step of calculating the surface to surface thermal conductance $\Lambda$, the surface to surface thermal conductance $\Lambda$ is calculated on the basis of a corrected value q' of heat flow;
- in the step of determining at least a first value $q_a$, each value of theat least a first value $q_a$ is determined by a first measurement of a respective series of the at least a series of measurements of heat flow;
- in the step of determining at least a second value $q_b$, each value of the at least a second value $q_b$ is determined by a second measurement of a respective series of the at least a series of measurements of heat flow;
- in the step of determining at least a value q, each value of the value q is determined by a measurement of a respective series of the at least a series of measurements of heat flow.

For the measurements of heat flow through a surface, it is possible to use a heat flow meter (13, 23), available on the market, or equivalent means for measurement, for example comprising a plate having known thermal properties and temperature sensors on the two sides of the plate.

Notwithstanding, the use of at least a heat flow meter (13, 23) simplifies the realisation of the system (100); it is therefore preferable that during the step of measuring a plurality of times at least a heat flow meter (13, 23) is used, each heat flow meter (13, 23) being applied on a respective surface of the lateral surfaces (S, S').

The above-discussed method reflects the more general teachings and the contribution of the present invention with respect to the prior art. The specific formulas used in the various steps for describing the heat transfer model are such as to fall within the knowledge of the expert in the sector; and the formula can vary should the simplifying hypotheses change.

For example, the formulas that will be introduced in the following for the case of a method that comprises only the step of maintaining $T_a$ and the step of maintaining $T_b$ can be generalised to the case of a method comprising n steps of maintaining at a determined temperature, with the following systems of equations, in the case of two series of measurements, whether of heat flow or of thermal power:

$$\begin{pmatrix} q_{1a} \\ q_{1b} \\ \vdots \\ q_{1n} \end{pmatrix} = \begin{bmatrix} (T_a - T_{1a}) & (T_a - T_{2a}) \\ (T_b - T_{1b}) & (T_b - T_{2b}) \\ \vdots & \vdots \\ (T_n - T_{1n}) & (T_n - T_{2n}) \end{bmatrix} \times \begin{pmatrix} H_{11} \\ H_{12} \end{pmatrix}$$

$$\begin{pmatrix} q_{2a} \\ q_{2b} \\ \vdots \\ q_{2n} \end{pmatrix} = \begin{bmatrix} (T_a - T_{1a}) & (T_a - T_{2a}) \\ (T_b - T_{1b}) & (T_b - T_{2b}) \\ \vdots & \vdots \\ (T_n - T_{1n}) & (T_n - T_{2n}) \end{bmatrix} \times \begin{pmatrix} H_{21} \\ H_{22} \end{pmatrix}$$

in which $H_{11}$, $H_{12}$, $H_{21}$ and $H_{22}$ are corrective coefficients, as will be more fully illustrated in the following. The equation systems can be solved using an optimisation method, for example, with the least squares method.

An example of a different simplifying hypothesis is given by the possibility of keeping the first outside temperature $T_{1e}$ constant and equal to that of internally of the first volume (10V) in the step of maintaining $T_c$ and $T_d$; this hypothesis could be considered valid in the case of locating the first hollow object (10) in a climate-conditioned ambient and setting the test temperature $T_c$ of the first volume (10V) equal to the first outside temperature $T_{1e}$.

The embodiment that comprises only the step of maintaining $T_a$ and the step of maintaining $T_b$ is more rapid and, possibly, enables measurement of the thermal capacitance (C) as will be illustrated in the following.

The method preferably comprises steps of:

- acquiring or determining representative values $T_{1a}$ and $T_{2a}$ of the trend over time of the first outside temperature $T_{1e}$ and of the second outside temperature $T_{2e}$ in the step of maintaining $T_a$ or in a temporal part of the step maintaining $T_a$;
- acquiring or determining representative values $T_{1b}$ and $T_{2b}$ of the trend over time of the first outside temperature $T_{1e}$ and of the second outside temperature $T_{2e}$ in the step of maintaining $T_b$ or in a temporal part of the step step of maintaining $T_b$ ;
- acquiring or determining representative values $T_1$ and $T_2$ of the trend over time of the first outside temperature $T_{1e}$ and of the second outside temperature $T_{2e}$ in the step of maintaining $T_c$ and $T_d$ or in a temporal part of the step step of maintaining $T_c$ and $T_d$.

By "acquiring" is meant a relative information, as might occur in the case of inserting the first outside temperature $T_{1e}$ set in a climate-conditioned ambient. Generally each of the representative values ($T_{1a}$, $T_{2a}$, $T_{1b}$, $T_{2b}$, $T_1$ and $T_2$) is determined by measurements over time and a calculation of the average time, so as to be representative of the forcing mechanism, given the difference of temperature, which has determined the three-dimensional effects of the heat flow that have an influence on the steps of determining at least a value q, a first value $q_a$ or a second value $q_b$ of heat flow or of thermal power.

Therefore:

- in the step of measuring a plurality of times,

    a heat flow through at least a part of a first surface (S) of the lateral surfaces (S, S') facing towards the first hollow object (10) is measured
    or, alternatively,
    a thermal power supplied by the at least a first heater and/or cooler (17, 17') is measured as well as a thermal power supplied by the at least a second heater and/or cooler (27, 27')
so as to obtain at least a first series of measurements of heat flow relative to a first surface (S) of the at least a series of measurements of heat flow or of the series of measurements of thermal power;

- in the step of calculating corrective coefficients, calculations are made of at least a first corrective coefficient $H_{11}$ with the formula

$$H_{11} = \frac{q_{1a}\,(T_b - T_{2b}) - q_{1b}\,(T_a - T_{2a})}{(T_a - T_{1a})(T_b - T_{2b}) - (T_a - T_{2a})(T_b - T_{1b})}$$

and a second corrective coefficient $H_{12}$ with the formula

$$H_{12} = \frac{q_{1b}\,(T_a - T_{1a}) - q_{1a}\,(T_b - T_{1b})}{(T_a - T_{1a})(T_b - T_{2b}) - (T_a - T_{2a})(T_b - T_{1b})}$$

in which $q_{1a}$ and $q_{1b}$ are determined by a first series;

- in the step of determining a corrected value q', a first corrected value is calculated $q_1'$ of a first value $q_1$ of the at least a value q of heat flow or of thermal power on the basis of the formula

$$q_1' = q_1 - H_{11}(T_c - T_1) - H_{12}(T_d - T_2)$$

in which the first value $q_1$ of the at least a value q is determined by the first series.

As already indicated, the measurement of the heat flow enables using only the corrective coefficients $H_{11}$ and $H_{12}$.

For a greater precision and especially in the case of measurement of thermal power, it is preferable also to calculate a third corrective coefficient $H_{21}$ and a fourth corrective coefficient $H_{22}$.

In this case:

- in the step of measuring a plurality of times,

   a heat flow is measured through at least a part of a second surface (S') of the lateral surfaces (S, S') facing towards the second hollow object (20)
   or, alternatively,
   a thermal power supplied by the at least a first heater and/or cooler (17, 17') is measured as well as a thermal power supplied by the at least a second heater and/or cooler (27, 27')
   so as to obtain a second series relative to a second surface (S') of the at least a series of measurements of heat flow or of the series of measurements of thermal power;

- in the step of calculating corrective coefficients, also calculated are a third corrective coefficient $H_{21}$ and a fourth corrective coefficient $H_{22}$ with the following formulas

$$H_{21} = \frac{q_{2a}\,(T_b - T_{2b}) - q_{2b}\,(T_a - T_{2a})}{(T_a - T_{1a})(T_b - T_{2b}) - (T_a - T_{2a})(T_b - T_{1b})}$$

$$H_{22} = \frac{q_{2b}\,(T_a - T_{1a}) - q_{2a}\,(T_b - T_{1b})}{(T_a - T_{1a})(T_b - T_{2b}) - (T_a - T_{2a})(T_b - T_{1b})}$$

in which
$q_{2a}$ and $q_{2b}$ are determined by the second series;

- in the step of determining a corrected value q', a second corrected value is calculated $q_2'$ of a second value $q_2$ of the at least a value q of heat flow or of thermal power on the basis of the formula

$$q'_2 = q_2 - H_{21}(T_c - T_1) - H_{22}(T_d - T_2)$$

in which the second value $q_2$ of the at least a value q is determined by the second series.

The first corrected value $q'_1$ and the second corrected value $q'_2$ can be compared to one another in order to obtain an indication of the accuracy of the measurement of the surface to surface thermal conductance $\Lambda$ and/or be both used in the step of determining at least a corrected value q' of heat flow or of thermal power. Typically, in the step of determining at least a corrected value q' of heat flow or of thermal power the first corrected value $q'_1$ and the second corrected value $q'_2$ are averaged.

As mentioned in the foregoing, the measurement of heat flow, possibly, with a heat flow meter (13, 23), enables the simplification of the procedure or the measurement system (100). Preferably, in the step of measuring a plurality of times, only one heat flow is measured through at least a part of a respective surface of the lateral surfaces (S, S'), i.e. either through the first surface (S) or through the second surface (S').

On the basis of the definition of surface to surface thermal conductance $\Lambda$, the calculation thereof requires knowing the absolute value of the corrected value q' of heat flow or the thermal power and the temperatures of the lateral surfaces (S, S'), as well as the area $A_s$ of the lateral surfaces (S, S') in the case of uses of the thermal power. Although the temperatures internally of the first volume (10V) and the second volume (20V) might validly represent the temperatures of the lateral surfaces, especially in the case of a very insulated opaque component (O), it is preferable to take the measurement thereof to reduce the error in the measurement.

The method preferably comprises a step of:

- measuring a respective surface temperature ($T_{s1}$, $T_{s2}$) for both the lateral surfaces (S, S') at the end of the step of maintaining $T_c$ and $T_d$.

In the step of calculating the surface to surface thermal conductance $\Lambda$, the surface to surface thermal conductance $\Lambda$ is calculated on the basis of the ratio between the absolute value of the corrected value q' of heat flow or of thermal power and the absolute value of the difference between the first surface temperature $T_{s1}$ and the second surface temperature $T_{s2}$.

For example, in the case of measurements of heat flow and average the following formula can be used:

$$\Lambda = \frac{0.5\,(|q'_1| + |q'_2|)}{|T_{s1} - T_{s2}|}$$

while in the case of measurements of thermal power and average the following formula can be used:

$$\Lambda = \frac{0.5\,(|q'_1| + |q'_2|)}{A_s\,|T_{s1} - T_{s2}|}.$$

In the field of the in situ measurements of the thermal properties of the opaque components, errors of measurements are particularly significant and even the instrumental errors are not irrelevant, as they can be of the order of 2%, while they are usually less than 5%.

In the steps of maintaining $T_c$ and $T_d$, of maintaining $T_a$ and of maintaining $T_b$ the stationary conditions are obtained in an infinite time. To take account of this aspect it is possible to wait for a predetermined time interval $t$, a first predetermined time interval $t_1$ and a second predetermined time interval $t_2$ that is sufficiently long and/or to take as a base the asymptotic trend of the measurement, though subject to fluctuations.

Preferably, in the steps of maintaining $T_c$ and $T_d$, of maintaining $T_a$ and of maintaining $T_b$ the respective desired value $d$, first desired value $d_1$ and second desired value $d_2$ is lower than 5% between two successive measurements distanced by time from one another between 15 and 45 minutes, more preferably 30 minutes.

If this condition is indicative of the reaching of an almost-stationary condition, the wait for a time interval $t$, a first time interval $t_1$ and a second time interval $t_2$ of at least 24 hours generally enables comprising the whole fluctuation of the external conditions due to the combination of day and night.

Preferably, each between the time interval $t$, the first time interval $t_1$ and the second time interval $t_2$ is greater than or equal to 24 hours.

Preferably, especially in the case mentioned above of time intervals of greater than or equal to 24 hours, in the steps of maintaining $T_a$, maintaining $T_b$ and of maintaining $T_c$ and $T_d$, the temperatures are maintained exclusively on the basis, respectively, of the first time interval $t_1$, of the second time interval $t_2$ and of the time interval $t$. In this way the management of the test can be simplified with a management of the wait that is sufficient to reach more or less the steady state condition and to keep acount of the main fluctuations of the outside ambient conditions.

The method described herein can also advantageously be used to calculate the thermal capacitance (C) of the volume (V) of opaque component (O) without particular complications, i.e. it enables better characterising the opaque component (O) in a simple way and substantially in a single sequence of operations. This is possible in the case of heat flow measurements.

Preferably:

- in the step of measuring a plurality of times, measurement is made continuously or at time intervals of heat flows through at least a part of both the lateral surfaces (S, S');
- the step of maintaining $T_b$ begins at the end of the step of maintaining $T_a$;
- the method comprises a step of:

  - calculating the thermal capacitance (C) of the volume (V) of opaque component (O) on the basis of the series of the at least a series of measurements of heat flow carried out between the start of the step of maintaining $T_b$ and when the variation between successive measurements of a series of the series of measurements of heat flow is lower than the second desired value or than a further desired value.

The further desired value is useful in a case of desiring a different value for the measurement of surface to surface thermal conductance $\Lambda$ and for the measurement of thermal capacitance (C), for example so as to first terminate the thermal capacitance (C) so as to reduce the external influences in the measurement.

More preferably, in order to take account of the variability of the heat flows within the step of maintaining $T_b$:

- in the step of measuring a plurality of times, at least in the step of maintaining $T_b$ the discrete time interval $\Delta t_i$ between two successive measurements is less than 30 minutes, and is generally constant;
- in the step of calculating the thermal capacitance (C), the thermal capacitance (C) is calculated with the following formula

$$C = \frac{\sum q_{1_i}^* \Delta t_i + \sum q_{2_i}^* \Delta t_i}{T_b - T_a}$$

in which

$\sum q_{1_i}^* \Delta t_i$ and $\sum q_{2_i}^* \Delta t_i$ are the sum of the energies through the respective surface of the lateral surfaces (S, S') in each discrete time interval $\Delta t_i$.

As already mentioned in the foregoing, it is generally preferable:

- to measure, continuously or at time intervals, not necessarily but preferably cadenced, the first outside temperature $T_{1e}$;
- to measure, continuously or at time intervals, not necessarily but preferably cadenced, the second outside temperature $T_{2e}$;

Further, in the steps of acquiring or determining representative values $T_{1a}$ and $T_{2a}$, acquiring or determining representative values $T_{1b}$ and $T_{2b}$ and acquiring or determining representative values $T_1$ and $T_2$, the representative values $T_{1a}$, $T_{2a}$, $T_{1b}$, $T_{2b}$, $T_1$ and $T_2$ are the time averages, of the first outside temperature ($T_{1e}$) or of the second outside temperature ($T_{2e}$) in the respective step or in a part thereof; in the case of measurements of thermal power, the part of the respective step preferably coincides with, respectively, the time interval, the first time interval and the second time interval, so that there is a correlation between the forcing mechanisms determined by the temperatures and the thermal power measured.

Ambient air (A, A') is present on both sides of the opaque component (O) externally of the first hollow object (10) and the second hollow object (20), i.e. the ambient air (A, A'), is opposite the first volume (10V) and the second volume (20) with respect to, respectively, the first hollow object (10) and the second hollow object (20).

The expert in the sector will understand that the first outside temperature $T_{1e}$ must be representative of the temperature of the first ambient air (A) which on one side of the opaque component (O) strikes the opaque component (O) externally of the first hollow object (10) and that the second outside temperature $T_{2e}$ must be representative of the temperature of the second ambient air (A') that, on the other side of the opaque component (O), strikes the opaque component (O) externally of the second hollow object (20).

Regarding the components necessary for the carrying out of the method, as well as for the carrying out of the method described in the following, reference is made to the knowledge of the expert in the sector as well as to the description of the components of the systems (100) herein below.

As regards the sequence between the steps, where this is not clear from the relations of the steps, the steps can be carried out in different orders, and possibly at the same time. Clearly the step of measuring a plurality of times is carried out at the same time as other steps while, for example, the step of calculating corrective coefficients might be at least partially contemporaneous to the step of maintaining $T_c$ and $T_d$.

No sequence is necessary between the steps of maintaining $T_a$, maintaining $T_b$ and maintaining $T_c$ and $T_d$, although typically the step of maintaining $T_c$ and $T_d$ follows the others. It is also preferable for the sequence to minimise the temperature variations in the first volume (10V) and in the second volume (20V) so as to reduce the energy demand of the measurement and facilitate a more rapid achieving of the almost-stationary conditions. From the above, it is clear that the step of maintaining $T_b$ follows the step of maintaining $T_a$ in a case where there is the step of calculating the thermal capacitance (C).

The above description has already shown how the teachings of the invention enable the measurement of the thermal capacitance (C); this measurement can also be the object of a method that is independent of the measurement of the surface to surface thermal conductance $\Lambda$.

The invention also relates to a method for in situ measurement of the thermal capacitance (C) of a volume (V) of an opaque component (O) of a building.

This method does not require all the above-described steps but in order to comprehend the description of the various embodiments reference can be made to the above description in relation to the steps of interest of the following embodiment.

An embodiment of the method comprises steps of:

- providing at least a first heater and/or cooler (17, 17');
- providing at least a second heater and/or cooler (27, 27');
- placing a first hollow object (10) and a second hollow object (20) on opposite sides of an opaque component (O) in such a way as to enclose lateral surfaces (S, S'), mutually opposite, of a volume (V) of an opaque component (O) and which delimit, together with the opaque component (O), respectively a first volume (10V) and a second volume (20V) heated and/or cooled respectively by the at least a first heater and/or cooler (17, 17') and by the at least a second heater and/or cooler (27, 27');
- measuring a heat flow, continuously or at time intervals, through at least a part of a first surface of the lateral surfaces (S, S') facing towards the first hollow object (10) and a heat flow through at least a part of a second surface of the lateral surfaces (S, S') facing towards the second hollow object (20) so as to obtain at least a first series of measurements of heat flow relative to a first surface (S) and a second series of measurements of heat flow relative to the second surface (S');
- maintaining $T_a$, maintaining the first volume (10V) and the second volume (20V) at a first test temperature $T_a$, equal between the first volume (10V) and the second volume (20V), for a first predetermined time interval $t_1$ and/or at least until when the variation between time-successive measurements of a series of the first series of measurements and the second series of measurements is lower than a first desired value $d_1$;
- maintaining $T_b$, maintaining the first volume (10V) and the second volume (20V) at a second test temperature $T_b$, equal between the first volume (10V) and the second volume (20V), until when the variation between time-successive measurements of a series of the first series of measurements and the second series of measurements is lower than second desired value $d_2$;
- calculating the thermal capacitance (C) of the volume (V) of opaque component (O) on the basis of the first series of measurements and of the second series of measurements carried out between the start and the end of the step of maintaining $T_b$;

and the step of maintaining $T_b$ begins at the end of the step of maintaining $T_a$.

It is therefore possible simply and relatively rapidly to determine an indication of the thermal capacitance of the opaque component (O).

Preferably, in order to take account of the variability of the heat flows within the step of maintaining $T_b$:

- in the step of measuring, continuously or at time intervals, the discrete time interval $\Delta t_i$ between two successive measurements, at least in the step of maintaining $T_b$, is less than 30 minutes, and is generally constant;
- in the step of calculating the thermal capacitance (C), the thermal capacitance (C) is calculated with the following formula

$$C = \frac{\sum q^*_{1_i}\Delta t_i + \sum q^*_{2_i}\Delta t_i}{T_b - T_a}$$

in which

$\sum q^*_{1_i}\Delta t_i$ and $\sum q^*_{2_i}\Delta t_i$ are the sum of the energies through the respective surface of the lateral surfaces (S, S') in each discrete time interval $\Delta t_i$.

The invention also relates to a computer (4) program which can be operated directly in situ or in a remote location, both in real-time with the step of repeatedly measuring and by receiving series of measurements.

An embodiment of the program comprises instructions for carrying out the steps of calculating corrective coefficients of the at least a value q, determining a corrected value q' and calculating the surface to surface thermal conductance $\Lambda$ and, possibly, calculating the thermal capacitance (C) of the method for the in situ measurement of the surface to surface thermal conductance $\Lambda$.

The program preferably comprises instructions for carrying out the step of calculating the thermal capacitance (C) of the method for in situ measurement of the thermal capacitance (C).

The program preferably comprises instructions for facilitating the carrying out of the steps of maintaining $T_a$, maintaining $T_b$ and, possibly, maintaining $T_c$ and $T_d$ configured in such a way as to provide indications on having maintained the temperatures with respect to the first time interval $t_1$, the second time interval $t_2$ and, possibly, the time interval $t$, and/or configured in such a way as to provide indications on the reaching of a variation between measurements lower than a first desired value $d_1$, to a second desired value $d_2$ and, possibly, to a desired value $d$. The program can comprise instructions for carrying out the steps of determining at least a value q, determining at least a first value $q_a$ and determining at least a second value $q_b$.

The invention also relates to a system (100) for measuring the surface to surface thermal conductance $\Lambda$ and/or the thermal capacitance (C) of a volume (V) of an opaque component (O) of a building.

An embodiment of the system (100) comprises a first unit (1), a second unit (2), one or more systems of acquisition of the detections (3) and at least a control unit (17a, 27a).

The first unit (1) comprises a first hollow object (10), in turn comprising at least a wall (11a), a seal (11b) and an opening (12), at least an air temperature sensor (15, 15'), at least a first heater and/or cooler (17, 17') and a heat flow meter (13).

The second unit (2) comprises a second hollow object (20), in turn comprising at least a second wall (21a), a second seal (21b) and a second opening (22), at least a second air temperature sensor (25, 25'), at least a second heater and/or cooler (27, 27') and a second heat flow meter (23).

The first unit (1) and the second unit (2) are on opposite sides of an opaque component (O).

The first hollow object (10) and the second hollow object (20) are on opposite sides of an opaque component (O) in such a way as to enclose lateral surfaces (S, S'), mutually opposite, of a volume (V) of an opaque component (O) and so as to delimit, together with the opaque component (O), respectively a first volume (10V) and a second volume (20V).

The seal (11b) surrounds the opening (12) and is interposed between the at least a wall (11a) and the opaque component (O) so as to guarantee the air seal between the at least a wall (11a) and the opaque component (O).

The second seal (21b) surrounds the second opening (22) and is interposed between the at least a second wall (21a) and the opaque component (O) so as to guarantee the air seal between the at least a second wall (21a) and the opaque component (O).

The at least an air temperature sensor (15, 15') is located internally of the first volume (10V).

The at least a second air temperature sensor (25, 25') is located internally of the second volume (20V).

The at least a first heater and/or cooler (17, 17') heats and/or cools the first volume (10V).

The at least a second heater and/or cooler (27, 27') heats and/or cools the second volume (20V).

The at least a control unit (17a, 27a) receives information from at least a sensor of the at least an air temperature sensor (15, 15') and from at least a sensor of the at least a second air temperature sensor (25, 25') and controls the at least a first heater and/or cooler (17, 17') and the at least a second heater and/or cooler (27, 27').

The heat flow meter (13) is applied on a first surface (S) and the second heat flow meter (23) is applied on a second surface (S), opposite the first surface (S), of the lateral surfaces (S, S') of the volume (V) of opaque component (O).

The above-described system (100) enables measurement of fundamental thermal properties for the characterisation of the opaque component, as it is able to measure both the thermal capacitance (C) and the surface to surface thermal conductance $\Lambda$, in the second case being able to supply the information necessary for the application of the method as described in the foregoing and therefore, for the calculation of corrective coefficients of the distorting effects of the ambient temperatures.

The application of the system (100), as well as the application of the method for in situ measurement of the surface to surface thermal conductance $\Lambda$, is particularly advantageous, with respect to the solutions in the prior art, with an increase of the layers of the stratigraphy and, especially, with a decrease in the thermal conductance.

With the application of the method for in situ measurement of the surface to surface thermal conductance $\Lambda$, the dimensions of the first hollow object (10) and of the second hollow object (20) can be reduced, without the three-dimensional aspect of the heat flow impacting on the final measurement or, in any case, having a minimum impact. The system (100) described above enables obtaining a precise measurement in a simple and non-destructive way. The system (100) is preferably used for carrying about the above-described methods.

The system (100) preferably comprises a computer (4) program as described in the foregoing. The system (100) more preferably comprises a computer (4), which carries out the program, but in an entirely equivalent way the program might be carried out remotely, for example on an external server.

As is clarified in the foregoing description the system (100) with heat flow meters (13, 23) is effective and also enables measuring the thermal capacitance (C) but also applicable is a system (100) which comprises other means for measurement, for example for measuring the thermal power. The foregoing includes a discussion of the convenience of the measurements of thermal power in terms of the simplification of the system (100) and of the possibility of measuring opaque components having lateral surfaces (S, S') very dissimilar to flat planes, i.e. with furrows or projections, or having shapes, depths and directions that are variable. An example of this condition is represented by opaque components made of exposed natural stone; with the use of measurements of thermal power it is possible to measure the surface to surface thermal conductance $\Lambda$ that is representative of the opaque component (O), as it is substantially an average of the behaviour of the lateral surfaces (S, S').

The invention also relates to a system (100) for carrying out the method for in situ measurement of the surface to surface thermal conductance $\Lambda$ of a volume (V) of an opaque component (O) of a building according to the present description.

Similarly to what is described in the foregoing, an embodiment of the system (100) comprises a first unit (1), a second unit (2), one or more systems of acquisition of the detections (3), at least a control unit (17a, 27a) and, further, a computer program for a computer (4) as illustrated in the foregoing.

The first unit (1) comprises a first hollow object (10) in turn comprising at least a wall (11a), a seal (11b) and an opening (12), at least an air temperature sensor (15, 15'), an air temperature sensor (16), - at least a first heater and/or cooler (17, 17) and first means for measuring a heat flow or a thermal power supplied by the at least a first heater and/or cooler (17, 17').

The second unit (2) comprises a second hollow object (20) comprising at least a second wall (21a), a second seal (21b) and a second opening (22), at least a second air temperature sensor (25, 25'), a second air temperature sensor (26) and at least a second heater and/or cooler (27, 27').

The first unit (1) and the second unit (2) are on opposite sides of an opaque component (O) with the first hollow object (10) facing towards the second hollow object (20) positioned in such a way as to enclose lateral surfaces (S, S'), mutually opposite, of a volume (V) of an opaque component (O) and so as to delimit, together with the opaque component (O), respectively a first volume (10V) and a second volume (20V).

The seal (11b) surrounds the opening (12) and is interposed between the at least a wall (11a) and the opaque component (O) so as to guarantee the air seal between the at least a wall (11a) and the opaque component (O).

The second seal (21b) surrounds the second opening (22) and is interposed between the at least a second wall (21a) and the opaque component (O) so as to guarantee the air seal between the at least a second wall (21a) and the opaque component (O).

The at least an air temperature sensor (15, 15') is located internally of the first volume (10V).

The air temperature sensor (16) is located externally of the first hollow object (10).

The at least a second air temperature sensor (25, 25') is located internally of the second volume (20V).

The second air temperature sensor (26) is located externally of the second hollow object (20).

The at least a first heater and/or cooler (17, 17') heats and/or cools the first volume (10V).

The at least a second heater and/or cooler (27, 27') heats and/or cools the second volume (20V).

The at least a control unit (17a, 27a) receives information from at least a sensor of the at least an air temperature sensor (15, 15') and from at least a sensor of the at least a second air temperature sensor (25, 25') and controls the at least a first heater and/or cooler (17, 17') and the at least a second heater and/or cooler (27, 27').

The first means measure a heat flow on the surface facing towards the first hollow object (10) of the mutually opposite lateral surfaces (S, S') of the volume (V) of opaque component (O) or measure a thermal power supplied by the at least a first heater and/or cooler (17, 17').

In the case where the first means measure the thermal power, the second unit comprises second means for measuring a thermal power supplied by the at least a second heater and/or cooler (27, 27').

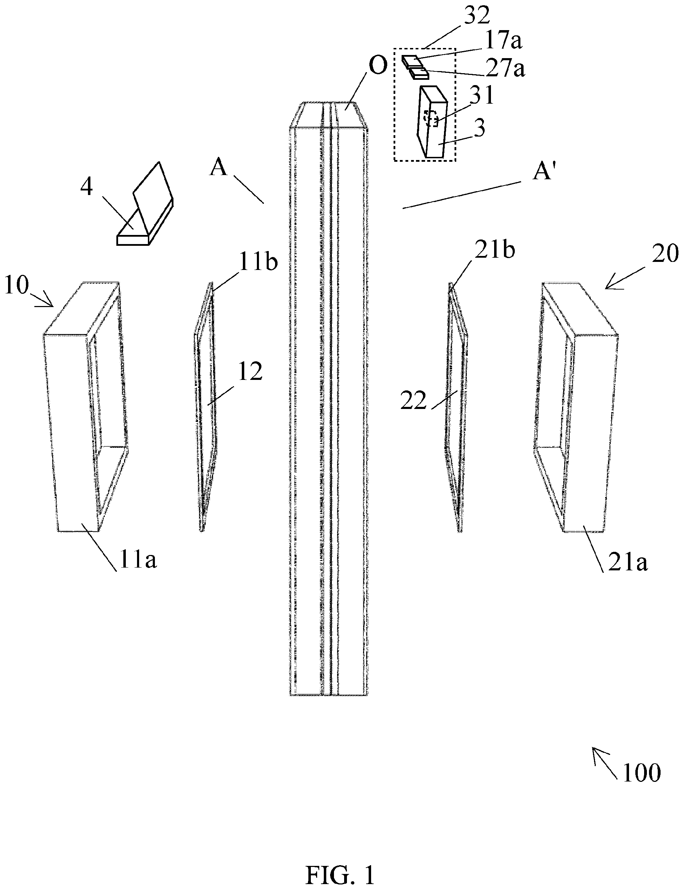

The system (100) can also comprise a computer (4) transportable in situ, as shown by way of example in figure 1, or the computer (4) program can be carried out remotely, possibly also at a later time with respect to the acquisition of the detections.

The preferred embodiments and considerations illustrated in the foregoing for the system (100) for measuring the surface to surface thermal conductance $\Lambda$ and/or the thermal capacitance (C) of a volume (V) of an opaque component (O) of a building are also applicable in the system (100) for measuring the surface to surface thermal conductance $\Lambda$ of a volume (V) of an opaque component (O) of a building.

The following contains details of further embodiments of the systems (100) for measuring introduced in the foregoing, including with particular reference to the examples in the appended figures of the drawings.

The first hollow object (10) and the second hollow object (20) should be dimensioned so as to guarantee stationary conditions of heat flow in the parts of the lateral surfaces (S, S') where the heat flow meters (13), (23) are applied, in the central part according to a preferred application mode.

In the case of the first volume (10V) or the second volume (20V) having a parallelepiped shape as in the appended figures the sides vary preferably between 0.6 m and 1.6 m with a thickness, i.e. the distance from the opaque component (O), comprised between 0.1 m and 0.4 m. Clearly, the more the dimensions reduce, the lower the energy demand during the measurement. With excessively reduced dimensions it might be particularly difficult to reach near-stationary conditions.

Regarding the appended figures, which illustrate embodiments of the parallelepiped first volume (10V) or the second volume (20V), the at least a wall (11a) might be singular, for example in the form of a hemisphere.

The systems (100) for measuring are described in the mounted condition, once applied to an opaque component (O) to facilitate the comprehension of the components thereof and their characteristics. This is in particular because in the case of use of heat flow meters (13, 23), they must be applied on the lateral surfaces (S, S') as they are, in general, the sensors of at least a surface temperature sensor (14, 14' 14") and of at least a second surface temperature sensor (24, 24', 24"), described in the following. In this regard, these components, for example the heat flow meters (13, 23), might be supported by the at least a wall (11a) and by the at least a second wall (21a) or, as usually happens, applied on the opaque component (O) in known ways, so as to guarantee perfect adhesion to the lateral surfaces (S, S'). As in general for all the methods introduced in the description of the prior art, the expert in the branch knows preparatory operations and application procedures for the apparatus required in order to ensure correct measurement. By way of example, standard ASTM C1046 defines the installation modality of the sensors for in situ measurement of the heat resistance of opaque structures of buildings and provides a guide on the use of infrared thermography for verifying the absence of disturbing elements, such as pipes or conduits, inside the wall and standard ASTM C1130 provides information on the calibration of the heat flow meters (13, 23).

In the same way, from the description of the mounted systems (100), the characteristics of the demounted systems (100) will be obvious. For example, with the aim of including the opposite lateral surfaces (S, S') of a volume (V) of an opaque component (O), i.e. with the aim of including surfaces between which an almost one-dimensional heat flow is maintained, it is necessary for the opening (12) and the second opening (22) to define a same area, the at least a wall (11a) and the at least a second wall (21a) are consequently located with respect to one another in the conditions given by way of example in figures from 9 to 11.

As illustrated in the appended figures, the first unit (1) preferably comprises at least a surface temperature sensor (14, 14', 14") and/or, more preferably, the second unit (2) comprises at least a second surface temperature sensor (24, 24', 24"). This enables controlling the measurement underway and arranging the first surface temperature $T_{s1}$ and the second surface temperature $T_{s2}$. Usually each sensor of the at least a surface temperature sensor (14, 14', 14") and of the at least a second surface temperature sensor (24, 24', 24") is in proximity of the respective heat flow meter (13, 23) but distanced therefrom so as to limit the influence on the measurement thereof; for example the distance is often between 6 and 15 cm, more preferably between 8 and 12 cm.

With the conditions as described above, the measurement should not be influenced by the effects of radiation, for example solar radiation. As well as appropriate procedures in the choice of the position of application, at least one from between the first unit (1) and the second unit (2) preferably, more preferably both, comprises a screen (18, 28) which is positioned so as to protect the opaque component (O) about the first seal (11b) and/or the second seal (21b) and, possibly, at least a part of the at least a wall (11a) or the at least a second wall (12a).

It is thus possible to limit the influence of the radiation on the heat flow that is created between the lateral surfaces (S, S') and, possibly, to simplify the control of the temperatures internally of the first volume (10V) and/or the second volume (20V).

The screen (18, 28) should preferably extend on each side of the first hollow object (10) or the second hollow object (20) over at least a quarter of the involved dimension; for example, it should extend over at least 0.25m per side in the case of the parallelepiped first hollow object (10) having a square base with a 1 m side, thus obtaining a screen (18, 28) which covers at least a square having a side of 1.5 m.

Regardless of the presence of the screen (18, 28), the first unit (1) typically comprises an air temperature sensor (16), located externally of the first hollow object (10) and the second unit (2) comprises a second air temperature sensor (26), located externally of the second hollow object (20).

The at least one from between the air temperature sensor (16) and the second air temperature sensor (26) is preferably positioned between the screen (18, 28) and, respectively, the at least a wall (11a) or the at least a second wall (21a).

This enables obtaining a more precise detection of the ambient conditions that have an influence on the volume (V) of an opaque component (O), i.e. of the ambient air (A, A') which strikes the opaque component (O) in proximity of the volume (V). In this case too these detections can be used for controlling the measurement, i.e. for verifying the absence of disturbances.

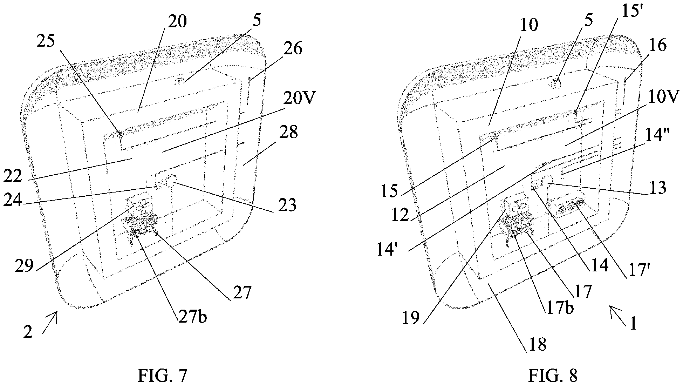

In the embodiments of figures 7 and 8 the screen (18, 28) comprises a respective rounded wall which extends from the wall of first hollow object (10) and of the second hollow object (20) opposite respectively to the opening (12) and to the second opening (22) up to a plane which is parallel to the opening (12) and to the second opening (22) and which cuts the respective first volume (10V) and the second volume (20V).

The at least one from between the first unit (1) and the second unit (2) preferably comprises a breather valve (5) which is configured so as to maintain the first volume (10V) and/or the second volume (20V) within a pressure range. In this way pressure variations are avoided, which might damage or break the components of the first unit (1) and the second unit (2) following the temperature variations internally of the first volume (10V) and the second volume (20V).

Typically both the at least a wall (11a) and the at least a second wall (21a) are insulated so as to limit the energy demand for maintaining the temperatures and for facilitating the control of the temperatures of the first volume (10V) and the second volume (20V).

At least one from between the at least a wall (11a) and the at least a second wall (21a) is preferably insulated so as to guarantee a heat dispersion through the walls thereof of less than 2 W/K, in the test conditions set for the system (100), i.e. taking into account the maximum difference of temperature between the first volume (10V) and the second volume (20V) and the respective external environment.

Especially in the case where the at least a wall (11a) or the at least a second wall (21a) are insulated, i.e. comprise insulating material or guarantee a heat dispersion of lower than 2 W/K, it is preferable that:

- the thickness of the first seal (11b) is smaller than the thickness or thicknesses of the at least a wall (11a) with which it is continuous; and/or
- the thickness of the second seal (21b) is smaller than the thickness or thicknesses of the at least a second wall (21a) with which it is continuous.

In this way there is a limiting of the distorting effects owing to transfer of heat energy by the heat flow between the lateral surfaces (S, S') towards the portions of opaque component (O) which are located between the contact areas between the opaque component (O) and, respectively, the first seal (11b) and the second seal (21b). This phenomenon, discussed in the foregoing, is visible in figures from 9 to 14 by observing the isotherms (T).

The thickness of the first seal (11b) and/or of the second seal (21b) is preferably constant, so as to avoid local distorsions of the heat flow in the opaque component (O).

In the functioning of the system (100) for measuring the surface to surface thermal conductance $\Lambda$ and/or the thermal capacitance (C), as well as in the step of maintaining $T_c$ and $T_d$, a heat flow is necessarily present so that one volume, from between the first volume (10V) and the second volume (20V), heats the other volume. The volume that is heated must therefore be in a condition such as to be able to dissipate the energy received towards the outside ambient. This can take place through the at least a wall (11a) or through the at least a second wall (21a), or by use of a cooler.

In a case where both the at least a wall (11a) and the at least a second wall (21a) are insulated, it is therefore preferable, though not necessary, for at least the at least a first heater and/or cooler (17, 17') and the at least a second heater and/or cooler (27, 27') to comprise a heater and a cooler.

Each of the at least a first heater and/or cooler (17, 17') and the at least a second heater and/or cooler (27, 27') preferably comprises an electrical resistance (17, 27), the advantages of which in terms of simplicity and controlability are known. The electrical resistance (17, 27) also enables easy detection of the thermal power provided on the basis of measurements of current. Further, each of the first unit (1) and second unit (2) preferably comprises a fan (19, 29) useful both for homogenising the temperature internally of the first volume (10V) and the second volume (20V), thus preventing stratification and for making the mixing between the two sides of the at least a wall (11a) and of the at least a second wall (21a) more alike.

A dissipater (17b, 27b) can also be present, for example between a respective electrical resistance (17, 27) an a respective fan (19, 29), as shown by way of example in the appended figures.

Each of the at least a first heater and/or cooler (17, 17') and the at least a second heater and/or cooler (27, 27') can comprise an internal unit (17', 27') to be connected to a refrigerating unit or a Peltier cell communicating with the outside environment.

Obviously the second unit (2) can comprise second means for measuring a heat flow.

In the case where the first means for measuring and/or the second means for measuring measure thermal powers, each thereof preferably comprises an amperometric sensor, and for measuring heat flows each thereof comprises a heat flow meter (13, 23).

Especially in the case of use of heat flow measurements, preferably the at least a surface temperature sensor (14, 14', 14") and the at least a second surface temperature sensor (24, 24', 24") both comprise two or more temperature sensors so as to verify the absence of disturbances in proximity of the heat flow meter (13, 23) and/or so as to be able to obtain an average value representative of the temperature of the opaque component where the heat flow meter (13, 23) is located.

The at least an air temperature sensor (15, 15') and the at least a second air temperature sensor (25, 25') can comprise a plurality of sensors, for example for redundancy or because one is used for detections useful for the measurement and the other for the control of the temperature internally of the first volume (10V) and the second volume (20V).

In figures from 9 to 11 the at least a control unit (17a, 27a) comprises at least a first unit (17a) and at least a second control unit (27a) but these might be integrated in a single control unit (17a, 27a).

Figure 1 illustrates a single system for acquisition of the detections (3) of a wireless type; clearly this might be of the cabled type and, in this case, it might be preferable to have available two distinct systems of acquisition of the detections (3) from the different sensors.

Generally, the cables which transport the live signals of the detections are conveyed internally of the at least a box (32) positioned externally of the first hollow object (10) and/or of the second hollow object (20) or the system (100) comprises communication devices without cables positioned internally of the at least a box (32). Typically the at least a box (32) is accessible by the technical expert who uses the system (100) during the measurement, with or without cable connection, using appropriate devices of known type. Analog/digital converters can be present internally of the at least a box (32) as well as the one or more systems of acquisition of the detections (3). Further, also at least a control unit (17a, 27a) is usually present in the at least a box (32).

Typically each of the one or more systems of acquisition of the detections (3) comprises a *datalogger* (31) for acquisition of the detections. Each *datalogger* (31) is connected, via a cable or cable-free, to one or more of the detection devices described in the present document, for example to the at least a surface temperature sensor (14, 14', 14").

The system (100) can comprise a suitable communication system in order to enable the monitoring and/or remote control, for example via the internet; the communication system can be located internally of the box (32).

The temperature sensors of the air or the surface temperature of the present invention can be heat resistances, i.e. *Resistance Temperature Detectors.*

Figures from 15 to 18 illustrate the models and application of the superposition principle of the effects that have led to the invention described herein. Figure 15 illustrates the step of maintaining $T_c$ and $T_d$ which, in working conditions that are approximately stationary, enables obtaining a one-dimensional heat flow, i.e. without edge effects. In figure 15 and in the following figures, the respective temperatures $T_c$ and $T_d$ are indicated as $T_1$ and $T_2$ so as to more clearly illustrate the demonstration and teachings of the invention. On the other hand, figure 16 illustrates the real condition, with the first outside temperature $T_{1e}$ and the second outside temperature $T_{2e}$ differing respectively from $T_c$ and $T_d$.

Although the behaviour of the opaque component (O) is very complex from the thermal point of view, in the stationary conditions it can be modelled with an electric circuit equivalent to a resistive circuit, like the one in figure 17. Figure 17 is a schematic illustration of the equivalent electric circuit of the opaque component (O) in which the voltage generators are illustrated, representing the forcing mechanisms of the system. The resistive circuit that simulates the behaviour of the opaque component (O) is enclosed in a black box. The voltage generators are three in number, one providing the difference between the temperatures ($T_c$ and $T_d$) between the first volume (10V) and the second volume (20V), one which provides the difference between the temperatures ($T_{1e}$ and $T_1$) on the side of the opaque component (O), where the first volume (10V) is located and one which provides the difference between the temperatures ($T_{2e}$ and $T_2$) on the side of the opaque component (O), where the second volume (20V) is located.

The functioning of the circuit of figure 17 can be reproduced using the superposition principle of the effects. The single effects are generated leaving one generator active at a time. The voltage generators that are switched off provide a short circuit. In each branch of the circuit, the sum of the currents in all the single effects will be equal to the current of the circuit of figure 17. The three equivalent circuits that generate the single effects are shown in figure 18, and of these only the one high on the left is the circuit of interest for measurement of the surface to surface thermal conductance $\Lambda$ as it does not suffer from the edge effects, i.e. the distorting effects.

In the experimental procedure, the heat flow meters (13, 23) located internally of the first volume (10V) and the second volume (20V) measure the heat flows obtained by the sum of the three effects. By denoting the heat flows detected in the first volume (10V) and the second volume (20V) of figure 15 respectively with $q_1^0$ and $q_2^0$, these can be written as follows:

$$q_1^0 = q_1^1 + q_1^2 + q_1^3$$

$$q_2^0 = q_2^1 + q_2^2 + q_2^3$$

in which the three contributions, distinguished by an apex are relative to the single effects of figure 18. The $q_1^1$ and $q_2^1$ are the heat flows of interest of the circuit high on the left in figure 18.

The flow $q_1^2$ represents the single contribution which starts from the first volume (10V) and reaches the outside environment on the same side as the opaque component (O). It depends on the difference in temperature between $T_1$ and $T_{1e}$ and can be expressed as:

$$q_1^2 = H_{11}(T_1 - T_{1e})$$

wherein $H_{11}$ is the reciprocal of the equivalent resistance that connects the two branches on the left of the circuit. The signs convention adopted has the heat flow as positive when entering in the opaque component (O). The same reasoning can be followed for the other heat flows, thus obtaining the following expressions:

$$q_1^0 = q_1^1 + H_{11}(T_1 - T_{1e}) + H_{12}(T_2 - T_{2e})$$

$$q_2^0 = q_2^1 + H_{21}(T_1 - T_{1e}) + H_{22}(T_2 - T_{2e})$$

In these equations, the two flows to the first member are directly measured, as well as the temperatures of the air. The four coefficients are unknown, given that the stratigraphy of the opaque component (O) is not known: they in fact represent heat transmissions which define the heat exchanges at edges due to the forcing mechanisms, i.e. to the first outside temperature $T_{1e}$ and to the second outside temperature $T_{2e}$. In order to determine the heat flows $q_1^1$ and $q_2^1$ without these effects, i.e. corrected, it is necessary to determine the four coefficients.

According to the teachings of the present invention, the four coefficients can be determined by carrying out experimental tests during which the values $q_2^1$ and $q_2^1$ are necessarily nil so that the detections of heat flow only provide the heat dispersions for the distorting effects. This condition occurs when the first volume (10V) and the second volume (20V) are maintained at the same temperatures, in the steady state condition; as mentioned in the foregoing at least a step of maintaining at $T_a$ and a step of maintaining at $T_b$ are necessary so as to obtain two equation systems in two decoupled unknowns which, by means of Kramer's theorem can be written as:

$$H_{11} = \frac{q_{1a}\,(T_b - T_{2b}) - q_{1b}\,(T_a - T_{2a})}{(T_a - T_{1a})(T_b - T_{2b}) - (T_a - T_{2a})(T_b - T_{1b})}$$

$$H_{12} = \frac{q_{1b}\,(T_a - T_{1a}) - q_{1a}\,(T_b - T_{1b})}{(T_a - T_{1a})(T_b - T_{2b}) - (T_a - T_{2a})(T_b - T_{1b})}$$

$$H_{21} = \frac{q_{2a}\,(T_b - T_{2b}) - q_{2b}\,(T_a - T_{2a})}{(T_a - T_{1a})(T_b - T_{2b}) - (T_a - T_{2a})(T_b - T_{1b})}$$

$$H_{22} = \frac{q_{2b}\,(T_{a1} - T_{1a}) - q_{2a}\,(T_{b1} - T_{1b})}{(T_a - T_{1a})(T_b - T_{2b}) - (T_a - T_{2a})(T_b - T_{1b})}.$$

Consequently, the heat flows $q_1^1$ and $q_2^1$ without these effects, i.e. corrected, can be calculated from the above expressions. The thus-measured values of $q_1^1$ and $q_2^1$ continue unequal for the further reasons that have been discussed in the foregoing, as well as for the errors in measurement.

## Claims

1. A method for in situ measurement of the surface to surface thermal conductance $\Lambda$ of a volume (V) of an opaque component (O) of a building comprising steps of:

- providing at least a first heater and/or cooler (17, 17');
- providing at least a second heater and/or cooler (27, 27');
- placing a first hollow object (10) and a second hollow object (20) on opposite sides of an opaque component (O) in such a way as to enclose lateral surfaces (S, S'), mutually opposite, of a volume (V) of an opaque component (O) and which delimit, together with the opaque component (O), respectively a first volume (10V) and a second volume (20V) heated and/or cooled respectively by the at least a first heater and/or cooler (17, 17') and by the at least a second heater and/or cooler (27, 27');
- measuring a plurality of times,

measuring at least a heat flow through at least a part of a respective surface of the lateral surfaces (S, S') so as to obtain at least a series of measurements of heat flow relative to a surface of the lateral surfaces (S, S') or, alternatively,

measuring a thermal power supplied by the at least a first heater and/or cooler (17, 17') and a thermal power supplied by the at least a second heater and/or cooler (27, 27'), so as to obtain series of measurements of thermal power supplied respectively to the first volume (10V) and to the second volume (20V);

- maintaining $T_c$ and $T_d$, maintaining the first volume (10V) and the second volume (20V) at a respective test temperature $T_c$ and $T_d$ different between the first volume (10V) and the second volume (20V), for a predetermined time interval $t$ and/or at least until when the variation between time-successive measurements of a series of the at least a series of measurements of heat flow or of a series of the series of measurements of thermal power is lower than a desired value $d$ ;
- determining at least a value q of heat flow or of thermal power with the first volume (10V) and the second volume (20V) at the respective test temperature $T_c$ and $T_d$, each value of the at least a value q being determined by a measurement, at the end of the step of maintaining $T_c$ and $T_d$, of a respective series of the at least a series of measurements of heat flow or by a representative value of the trend over time of measurements of a respective series of the series of measurements of thermal power in a time interval, within the step of maintaining $T_c$ and $T_d$,

the method being **characterised in that** it comprises steps of:

- maintaining $T_a$, maintaining the first volume (10V) and the second volume (20V) at a first test temperature $T_a$, equal between the first volume (10V) and the second volume (20V), for a first predetermined time interval $t_1$ and/or at least until when the variation between time-successive measurements of a series of the at least a series of measurements of heat flow or of a series of the series of measurements of thermal power is lower than a first desired value $d_1$;
- determining at least a first value $q_a$ of heat flow or of thermal power with the first volume (10V) and the second volume (20V) at the first test temperature $T_a$, each value of the at least a first value $q_a$ being determined by a first measurement, at the end of the step of maintaining $T_a$, of a respective series of the at least a series of measurements of heat flow or by a first representative value of the trend over time of measurements of a respective series of the series of measurements of thermal power in a first time interval, within the step of maintaining $T_a$;
- maintaining $T_b$, maintaining the first volume (10V) and the second volume (20V) at a second test temperature $T_b$, equal between the first volume (10V) and the second volume (20V), for a second predetermined time interval $t_2$ and/or at least until when the variation between time-successive measurements of a series of the at least a series of measurements of heat flow or of a series of the series of measurements of thermal power is lower than a second desired value $d_2$;
- determining at least a second value $q_b$ of heat flow or of thermal power with the first volume (10V) and the second volume (20V) at the second test temperature $T_b$, each value of the at least a second value $q_b$ being determined by a second measurement, at the end of the step of maintaining $T_b$, of a respective series of the at least a series of measurements of heat flow or by a second representative value of the trend over time of measurements of a respective series of the series of measurements of thermal power in a second time interval, within the step of maintaining $T_b$;
- calculating corrective coefficients of the at least a value q of heat flow or of thermal power, on the basis of the at least a first value $q_a$ and of the at least a second value $q_b$, the corrective coefficients correcting the effects of a first outside temperature $T_{1e}$ at the side of the opaque component (O) where the first hollow object (10) is placed and externally of the first hollow object (10) and the effects of a second outside temperature $T_{2e}$ at the side of the opaque component (O) where the second hollow object (20) is placed and externally of the second hollow object (20);
- determining a corrected value q' of heat flow or of thermal power with the first volume (10V) and the second volume (20V) at the respective test temperature $T_c$ and $T_d$ on the basis of the at least a value q of heat flow or of thermal power and of the corrective coefficients;
- calculating the surface to surface thermal conductance $\Lambda$ using the corrected value q' of heat flow or of thermal power.

2. The method of the preceding claim, wherein:

- in the step of measuring a plurality of times, at least a heat flow is measured through at least a part of a respective surface of the lateral surfaces (S, S') so as to obtain at least a series of measurements of heat flow relative to a surface of the lateral surfaces (S, S');
- in the step of calculating the surface to surface thermal conductance $\Lambda$, the surface to surface thermal conductance $\Lambda$ is calculated on the basis of a corrected value q' of heat flow;
- in the step of determining at least a first value $q_a$, each value of the at least a first value $q_a$ is determined by a first measurement of a respective series of the at least a series of measurements of heat flow;
- in the step of determining at least a second value $q_b$, each value of the at least a second value $q_b$ is determined by a second measurement of a respective series of the at least a series of measurements of heat flow;
- in the step of determining at least a value q, each value of the value q is determined by a measurement of a respective series of the at least a series of measurements of heat flow.

3. The method of the preceding claim wherein in the step of measuring a plurality of times at least a heat flow meter (13, 23) is used, each heat flow meter (13, 23) being applied on a respective surface of the lateral surfaces (S, S').

4. The method of any one of the preceding claims, comprising steps of:

- acquiring or determining representative values $T_{1a}$ and $T_{2a}$ of the trend over time of the first outside temperature $T_{1e}$ and of the second outside temperature $T_{2e}$ in the step of maintaining $T_a$ or in a part of the step of maintaining $T_a$;
- acquiring or determining representative values $T_{1b}$ and $T_{2b}$ of the trend over time of the first outside temperature $T_{1e}$ and of the second outside temperature $T_{2e}$ in the step of maintaining $T_b$ or in a part of the step of maintaining $T_b$;
- acquiring or determining representative values $T_1$ and $T_2$ of the trend over time of the first outside temperature $T_{1e}$ and of the second outside temperature $T_{2e}$ in the step of maintaining $T_c$ and $T_d$ or in a part of the step of maintaining $T_c$ and $T_d$;

wherein:

- in the step of measuring a plurality of times, a heat flow is measured through at least a part of a first surface of the lateral surfaces (S, S') facing towards the first hollow object (10) or, alternatively, a thermal power supplied by the at least a first heater and/or cooler (17, 17') is measured as well as a thermal power supplied by the at least a second heater and/or cooler (27, 27');
- in the step of calculating corrective coefficients, calculations are made of at least a first corrective coefficient $H_{11}$ with the formula

$$H_{11} = \frac{q_{1a}\,(T_b - T_{2b}) - q_{1b}\,(T_a - T_{2a})}{(T_a - T_{1a})(T_b - T_{2b}) - (T_a - T_{2a})(T_b - T_{1b})}$$

and of a second corrective coefficient $H_{12}$ with the formula

$$H_{12} = \frac{q_{1b}\,(T_a - T_{1a}) - q_{1a}\,(T_b - T_{1b})}{(T_a - T_{1a})(T_b - T_{2b}) - (T_a - T_{2a})(T_b - T_{1b})}$$

in which $q_{1a}$ and $q_{1b}$ are determined by a first series, relative to the first surface (S), of the at least a series of measurements of heat flow or of the series of measurements of thermal power;

- in the step of determining at least a corrected value q', a first corrected value $q_1'$ of a first value $q_1$ of the at least a heat flow value q or of thermal power is calculated on the basis of the formula

$$q'_1 = q_1 - H_{11}(T_c - T_1) - H_{12}(T_d - T_2)$$

in which the first value $q_1$ of the at least a value q is determined by the first series.

5. The method of the preceding claim, wherein:

- in the step of measuring a plurality of times, a heat flow is measured through at least a part of a second surface (S') of the lateral surfaces (S, S') facing towards the second hollow object (20) or, alternatively, a thermal power supplied by the at least a first heater and/or cooler (17, 17') is measured as well as a thermal power supplied by the at least a second heater and/or cooler (27, 27');
- in the step of calculating corrective coefficients, also calculated are a third corrective coefficient $H_{21}$ and a fourth corrective coefficient $H_{22}$ with the following formulas

$$H_{21} = \frac{q_{2a}(T_b - T_{2b}) - q_{2b}(T_a - T_{2a})}{(T_a - T_{1a})(T_b - T_{2b}) - (T_a - T_{2a})(T_b - T_{1b})}$$

$$H_{22} = \frac{q_{2b}(T_a - T_{1a}) - q_{2a}(T_b - T_{1b})}{(T_a - T_{1a})(T_b - T_{2b}) - (T_a - T_{2a})(T_b - T_{1b})}$$

in which
$q_{2a}$ and $q_{2b}$ are determined by a second series, relative to the second surface (S'), of the at least a series of measurements of heat flow or of the series of measurements of thermal power;

- in the step of determining a corrected value q', a second corrected value $q'_2$ of a second value $q_2$ of the at least a value q of heat flow or of thermal power is calculated on the basis of the formula

$$q'_2 = q_2 - H_{21}(T_c - T_1) - H_{22}(T_d - T_2)$$

in which the second value $q_2$ of the at least a value q is determined by the second series.

6. The method of claim 2 or 3 or of claim 4, when dependent on claim 2 or 3, wherein in the step of repeatedly measuring, measurement is made only of a heat flow through at least a part of a respective surface of the lateral surfaces (S, S').

7. The method of any one of the preceding claims, comprising steps of:

- measuring a respective surface temperature ($T_{s1}$, $T_{s2}$) for both the lateral surfaces (S, S') at the end of the step of maintaining $T_c$ and $T_d$;

and wherein in the step of calculating the surface to surface thermal conductance $\Lambda$, the surface to surface thermal conductance $\Lambda$ is calculated on the basis of the ratio between the absolute value of the corrected value q' of heat flow or of thermal power and the absolute value of the difference between the first surface temperature $T_{s1}$ and the second surface temperature $T_{s2}$.

8. The method of any one of the preceding claims, wherein in the steps of maintaining $T_c$ and $T_d$, of maintaining $T_a$ and of maintaining $T_b$ the respective desired value $d$, first desired value $d_1$ and second desired value $d_2$ is lower than 5% between two successive measurements distanced by time from one another between 15 and 45 minutes.

9. The method of claim 2 or one of the claims dependent thereon, wherein:

- in the step of measuring a plurality of times, measurement is made continuously or at time intervals of heat flows through at least a part of both the lateral surfaces (S, S');
- the step of maintaining $T_b$ begins at the end of the step of maintaining $T_a$;
- the method comprises a step of:

- calculating the thermal capacitance (C) of the volume (V) of opaque component (O) on the basis of the series of the at least a series of measurements of heat flow carried out between the start of the step of maintaining $T_b$ and when the variation between successive measurements of a series of the series of measurements of heat flow is lower than the second desired value $d_2$ or than a further desired value.

10. A method for in situ measurement of the thermal capacitance C of a volume (V) of an opaque component (O) of a building comprising steps of:

- providing at least a first heater and/or cooler (17, 17');
- providing at least a second heater and/or cooler (27, 27');
- placing a first hollow object (10) and a second hollow object (20) on opposite sides of an opaque component (O) in such a way as to enclose lateral surfaces (S, S'), mutually opposite, of a volume (V) of an opaque component (O) and which delimit, together with the opaque component (O), respectively a first volume (10V) and a second volume (20V) heated and/or cooled respectively by the at least a first heater and/or cooler (17, 17') and by the at least a second heater and/or cooler (27, 27');
- measuring a heat flow, continuously or at time intervals, through at least a part of a first surface of the lateral surfaces (S, S') facing towards the first hollow object (10) and a heat flow through at least a part of a second surface of the lateral surfaces (S, S') facing towards the second hollow object (20) so as to obtain at least a first series of measurements of heat flow relative to a first surface (S) and a second series of measurements of heat flow relative to the second surface (S');
- maintaining $T_a$, maintaining the first volume (10V) and the second volume (20V) at a first test temperature $T_a$, equal between the first volume (10V) and the second volume (20V), for a first predetermined time interval $t_1$ and/or at least until when the variation between time-successive measurements of a series of the first series of measurements and the second series of measurements is lower than a first desired value $d_1$;
- maintaining $T_b$, maintaining the first volume (10V) and the second volume (20V) at a second test temperature $T_b$, equal between the first volume (10V) and the second volume (20V), until when the variation between time-successive measurements of a series of the first series of measurements and the second series of measurements is lower than a second desired value $d_2$;
- calculating the thermal capacitance (C) of the volume (V) of opaque component (O) on the basis of the first series of measurements and of the second series of measurements carried out between the start and the end of the step of maintaining $T_b$;

wherein the step of maintaining $T_b$ begins at the end of the step of maintaining $T_a$.

11. A computer (4) program for measuring the surface to surface thermal conductance $\Lambda$ and/or the thermal capacitance (C) of a volume (V) of an opaque component (O) of a building comprising instructions so as to carry out the steps of calculating corrective coefficients of the at least a value q, determine at least a corrected value q' and calculate the surface to surface thermal conductance $\Lambda$ of the method of any one of claims 1 to 9.

12. A system (100) for measuring the surface to surface thermal conductance $\Lambda$ and/or the thermal capacitance (C) of a volume (V) of an opaque component (O) of a building comprising a first unit (1), a second unit (2), one or more systems of acquisition of the detections (3) and at least a control unit (17a, 27a), wherein:

- the first unit (1) comprises

- a first hollow object (10) comprising at least a wall (11a), a seal (11b) and an opening (12),
- at least an air temperature sensor (15, 15'),
- at least a first heater and/or cooler (17, 17),
- a heat flow meter (13);

- the second unit (2) comprises

- a second hollow object (20) comprising at least a second wall (21a), a second seal (21b) and a second opening (22),
- at least a second air temperature sensor (25, 25'),
- at least a second heater and/or cooler (27, 27'),
- a second heat flow meter (23);

wherein:

- the first unit (1) and the second unit (2) are on opposite sides of an opaque component (O) with the first hollow object (10) facing towards the second hollow object (20) positioned in such a way as to enclose lateral surfaces (S, S'), mutually opposite, of a volume (V) of an opaque component (O) and so as to delimit, together with the opaque component (O), respectively a first volume (10V) and a second volume (20V);

- the seal (11b) surrounds the opening (12) and is interposed between the at least a wall (11a) and the opaque component (O) so as to guarantee the air seal between the at least a wall (11a) and the opaque component (O);

- the second seal (21b) surrounds the second opening (22) and is interposed between the at least a second wall (21a) and the opaque component (O) so as to guarantee the air seal between the at least a second wall (21a) and the opaque component (O);

- the at least an air temperature sensor (15, 15') is located internally of the first volume (10V);

- the at least a second air temperature sensor (25, 25') is located internally of the second volume (20V);

- the at least a first heater and/or cooler (17, 17') heats and/or cools the first volume (10V);

- the at least a second heater and/or cooler (27, 27') heats and/or cools the second volume (20V);

- the at least a control unit (17a, 27a) receives information from at least a sensor of the at least an air temperature sensor (15, 15') and from at least a sensor of the at least a second air temperature sensor (25, 25') and controls the at least a first heater and/or cooler (17, 17') and the at least a second heater and/or cooler (27, 27');

- the heat flow meter (13) is applied on a first surface (S) and the second heat flow meter (23) is applied on a second surface (S), opposite the first surface (S), of the lateral surfaces (S, S') of the volume (V) of opaque component (O).

13. The system (100) of the preceding claim, wherein at least one from between the at least a wall (11a) and the at least a second wall (21a) is insulated so as to guarantee a heat dispersion through the walls thereof of less than 2 W/K.

14. The system (100) of the preceding claim, wherein both the at least a wall (11a) and the at least a second wall (21a) are insulated and the at least a second heater and/or cooler (27, 27') comprises a heater and a cooler.

15. A system (100) for carrying out the method for in situ measurement of the surface to surface thermal conductance $\Lambda$ of a volume (V) of an opaque component (O) of a building according to any one of claims 1 to 9 comprising a first unit (1), a second unit (2), one or more systems of acquisition of the detections (3), at least a control unit (17a, 27a) and a computer program for a computer (4) according to claim 11, wherein:

    - the first unit (1) comprises

        - a first hollow object (10) comprising at least a wall (11a), a seal (11b) and an opening (12),
        - at least an air temperature sensor (15, 15'),
        - an air temperature sensor (16),
        - at least a first heater and/or cooler (17, 17),
        - first means for measuring a heat flow or a thermal power supplied by the at least a first heater and/or cooler (17, 17');

    - the second unit (2) comprises

        - a second hollow object (20) comprising at least a second wall (21a), a second seal (21b) and a second opening (22),
        - at least a second air temperature sensor (25, 25'),
        - a second air temperature sensor (26),
        - at least a second heater and/or cooler (27, 27');

    wherein:

    - the first unit (1) and the second unit (2) are on opposite sides of an opaque component (O) with the first hollow object (10) and the second hollow object (20) located in such a way as to enclose lateral surfaces (S, S') that are opposite one another of a volume (V) of an opaque component (O) and so as to delimit, together with the opaque component (O), respectively a first volume (10V) and a second volume (20V);

    - the seal (11b) surrounds the opening (12) and is interposed between the at least a wall (11a) and the opaque component (O) so as to guarantee the air seal between the at least a wall (11a) and the opaque component (O);

    - the second seal (21b) surrounds the second opening (22) and is interposed between the at least a second wall (21a) and the opaque component (O) so as to guarantee the air seal between the at least a second wall (21a) and the opaque component (O);

- the at least an air temperature sensor (15, 15') is located internally of the first volume (10V);
- the air temperature sensor (16) is located externally of the first hollow object (10);
- the at least a second air temperature sensor (25, 25') is located internally of the second volume (20V);
- the second air temperature sensor (26) is located externally of the second hollow object (20);
- the at least a first heater and/or cooler (17, 17') heats and/or cools the first volume (10V);
- the at least a second heater and/or cooler (27, 27') heats and/or cools the second volume (20V);
- the at least a control unit (17a, 27a) receives information from at least a sensor of the at least an air temperature sensor (15, 15') and from at least a sensor of the at least a second air temperature sensor (25, 25') and controls the at least a first heater and/or cooler (17, 17') and the at least a second heater and/or cooler (27, 27');
- the first means measure a heat flow on the surface facing towards the first hollow object (10) of the mutually opposite lateral surfaces (S, S') of the volume (V) of opaque component (O) or measure a thermal power supplied by the at least a first heater and/or cooler (17, 17');
- in the case where the first means measure the thermal power, the second unit comprises second means for measuring a thermal power supplied by the at least a second heater and/or cooler (27, 27').

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

$T_1$ $T_2$

$T_1$ $T_2$

10V 20V

O

FIG. 15

$T_{1e}$ $T_{2e}$

$T_1$ $T_2$

10V 20V

O

FIG.16

$T_{1e}$ $T_{2e}$

$T_{1e}$-$T_1$ $T_1$ $T_2$ $T_{2e}$-$T_2$

$T_1$-$T_2$

FIG. 17

FIG. 18

EUROPEAN SEARCH REPORT

| Application Number |
| --- |
| EP 24 20 9142 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | WO 2016/150856 A1 (SENICO LTD [GB]) 29 September 2016 (2016-09-29) * page 5, line 28 - page 9, line 30 * | 1-15 | INV. G01N25/18 G01N33/00 |
| A | Nelson Soares: "Laboratory and in-situ non-destructive methods to evaluate the thermal transmittance and behavior of walls, windows, and construction elements with innovative materials: A review", Energy and buildings, 1 January 2019 (2019-01-01), pages 88-110, XP093157152, Lausanne DOI: 10.1016/j.enbuild.2018.10.021 Retrieved from the Internet: URL:https://www.sciencedirect.com/science/article/pii/S037877881831836X?via%3Dihub * page 27; figure 9 * | 1-15 | |
| A | MENG XI ET AL: "A new simple method to measure wall thermal transmittance in situ and its adaptability analysis", APPLIED THERMAL ENGINEERING, PERGAMON, OXFORD, GB, vol. 122, 16 May 2017 (2017-05-16), pages 747-757, XP085048416, ISSN: 1359-4311, DOI: 10.1016/J.APPLTHERMALENG.2017.05.074 * page 750, paragraph 3; figure 2 * | 1-15 | TECHNICAL FIELDS SEARCHED (IPC) G01N |

-/--

The present search report has been drawn up for all claims

| Place of search | Date of completion of the search | Examiner |
| --- | --- | --- |
| The Hague | 4 March 2025 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 24 20 9142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALBERTO BARBARESI: "Development of a low-cost movable hot box for a preliminary definition of the thermal conductance of building envelopes", BUILDING AND ENVIRONMENT, vol. 180, 1 August 2020 (2020-08-01), page 107034, XP093157216, GB ISSN: 0360-1323, DOI: 10.1016/j.buildenv.2020.107034 * page 3, paragraph 2.1 * | 1-15 | |
| A | TULLIO DE RUBEIS: "Integrated Measuring and Control System for Thermal Analysis of Buildings Components in Hot Box Experiments", ENERGIES, vol. 12, no. 11, 29 May 2019 (2019-05-29), page 2053, XP093157229, CH ISSN: 1996-1073, DOI: 10.3390/en12112053 * page 6; figure 2 * | 1-15 | |
| A | WO 2013/059007 A1 (CAMBRIA LTD [NZ]; THRESHER WAYNE CARL [NZ]; MCKINNON CLINTON [NZ]) 25 April 2013 (2013-04-25) * paragraph [0022]; figure 1 * | 1-15 | **TECHNICAL FIELDS SEARCHED (IPC)** |
| A,D | WO 2012/049417 A2 (INST FRANCAIS DES SCIENCES ET TECHNOLOGIES DES TRANSPORTS DE LAMENAGEM) 19 April 2012 (2012-04-19) * the whole document * | 1-15 | |
| A | CN 201 673 133 U (GUANGDONG RES INST OF CONSTRUCTION SCIENCES) 15 December 2010 (2010-12-15) * abstract * | 1-15 | |

The present search report has been drawn up for all claims

| Place of search | Date of completion of the search | Examiner |
|---|---|---|
| The Hague | 4 March 2025 | Joyce, David |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 9142

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2025

| Patent document cited in search report | | Publication date | Patent family member(s) | | Publication date |
|---|---|---|---|---|---|
| WO 2016150856 | A1 | 29-09-2016 | EP | 3274679 A1 | 31-01-2018 |
| | | | GB | 2536702 A | 28-09-2016 |
| | | | WO | 2016150856 A1 | 29-09-2016 |
| WO 2013059007 | A1 | 25-04-2013 | EP | 2769190 A1 | 27-08-2014 |
| | | | US | 2014286373 A1 | 25-09-2014 |
| | | | WO | 2013059007 A1 | 25-04-2013 |
| WO 2012049417 | A2 | 19-04-2012 | EP | 2627996 A2 | 21-08-2013 |
| | | | FR | 2965922 A1 | 13-04-2012 |
| | | | WO | 2012049417 A2 | 19-04-2012 |
| CN 201673133 | U | 15-12-2010 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20200101727 A
- EP 2769190 A4
- WO 2012049417 A2
- IT 201700148950
- IT 202000001282
- WO 2016150856 A1

**Non-patent literature cited in the description**

- **ZARR, R.R ; BURCH, D.M ; FAISON, T.K. ; ARNOLD, C.E**. Thermal Resistance Measurements of Well-Insulated and Superinsulated Residential Walls Using a Calibrated Hot Box. *Journal of Thermal Insulation.*, 1987, vol. 10 (3), 197-218
- **CUCUMO, M. ; DE ROSA, A. ; FERRARO, V. ; KALIAKATSOS, D. ; MARINELLI, V.** A method for the experimental evaluation in-situ of the wall conductance.. *Energy and Buildings*, 2006, vol. 38 (3), 238-244, https://doi.org/10.1016/J.EN-BUILD.2005.06.005
- **NICOLETTI, F. ; CUCUMO, M. ; ARCURI, N.** Evaluating the Accuracy of In-situ Methods for Measuring Wall Thermal Conductance: A Comparative Numerical Study. *Energy and Buildings*, 2023, 113095, https://doi.org/10.1016/J.EN-BUILD.2023.113095